# EUROPÄISCHE PATENTSCHRIFT

(11) **EP 0 889 886 B1**
(45) Veröffentlichungstag und Bekanntmachung des Hinweises auf die Patenterteilung: **18.09.2002**
(21) Anmeldenummer: 97915422.6
(22) Anmeldetag: 24.03.1997
(51) Int. Cl.: C07D 401/04, A61K 31/435, C07D 491/04, C07D 221/12

(54) **NEUE IN 6-POSITION SUBSTITUIERTE PHENANTHRIDINE**
NOVEL PHENANTHRIDINES SUBSTITUTED IN THE 6 POSITION
NOUVELLES PHENANTHRIDINES SUBSTITUEES EN POSITION 6

(30) Priorität: 26.03.1996 DE 19611922; 29.03.1996 EP 96105038; 02.04.1996 DE 19613091; 03.04.1996 EP 96105311
(43) Veröffentlichungstag der Anmeldung: 13.01.1999
(73) Patentinhaber: ALTANA Pharma AG, 78467 Konstanz (DE)
(72) Erfinder: AMSCHLER, Hermann, D-78315 Radolfzell (DE); FLOCKERZI, Dieter, D-78476 Allensbach (DE); ULRICH, Wolf-Rüdiger, D-78464 Konstanz (DE); BÄR, Thomas, D-78479 Konstanz (DE); MARTIN, Thomas, D-78462 Konstanz (DE); SCHUDT, Christian, D-78462 Konstanz (DE); HATZELMANN, Armin, D-78467 Konstanz (DE); BEUME, Rolf, D-78465 Konstanz (DE); HÄFNER, Dietrich, D-78464 Konstanz (DE); BOSS, Hildegard, D-78476 Allensbach (DE); KLEY, Hans-Peter, D-78476 Allensbach (DE); GUTTERER, Beate, D-78476 Allensbach (DE)
(86) Internationale Anmeldenummer: EP9701487
(87) Internationale Veröffentlichungsnummer: WO97035854

(56) Entgegenhaltungen:
- EP-A- 0 045 171
- FR-A- 2 144 609
- CHEMISCHE BERICHTE, Bd. 103, Nr. 6, 1970, WEINHEIM DE, Seiten 1674-1691, XP002009975 ERNST SEEGER ET AL: "Synthese 3.3-dialkylsubstituierter 3.4-Dihydro- und 1.2.3.4-Tetrahydro-isochinoline"
- JOURNAL OF LIQUID CHROMATOGRAPHY, Bd. 13, Nr. 3, 1.Januar 1990, Seiten 543-555, XP000196246 MORIYASU M ET AL: "A semicontinuous assay of inhibition of cyclic-amp phosphodiesterase by benzo[c]phenanthridine alkaloids"
- BERICHTE DER DEUTSCHEN CHEMISCHEN GESELLSCHAFT, Bd. 4, 1.Januar 1939, Seiten 675-678, XP000196051 SUGASAWA S ET AL: "Synthese partiell hydrierter Phenanthridin-Derivate" in der Anmeldung erwähnt

## Beschreibung

### Anwendungsgebiet der Erfindung

Die Erfindung betrifft neue in 6-Position substituierte Phenanthridine, die in der pharmazeutischen Industrie zur Herstellung von Medikamenten verwendet werden.

### Bekannter technischer Hintergrund

In Chem.Ber. **1939**, 72, 675-677, J.Chem.Soc., **1956**, 4280-4283 und J.Chem.Soc.(C), **1971**, 1805 wird die Synthese von 6-Phenylphenanthridinen beschrieben.

### Beschreibung der Erfindung

Es wurde nun gefunden, daß die nachfolgend näher beschriebenen neuen in 6-Position substituierten Phenanthridine überraschende und besonders vorteilhafte Eigenschaften besitzen.

Gegenstand der Erfindung sind somit Verbindungen der Formel I (siehe beigefügtes Formelblatt), worin
- R1: Hydroxy, 1-4C-Alkoxy, 3-7C-Cycloalkoxy, 3-7C-Cycloalkylmethoxy oder ganz oder teilweise durch Fluor substituiertes 1-4C-Alkoxy bedeutet,
- R2: Hydroxy, 1-4C-Alkoxy, 3-7C-Cycloalkoxy, 3-7C-Cycloalkylmethoxy oder ganz oder teilweise durch Fluor substituiertes 1-4C-Alkoxy bedeutet.
oder worin
- R1 und R2: gemeinsam eine 1-2C-Alkylendioxygruppe bedeuten,
- R3: Wasserstoff oder 1-4C-Alkyl bedeutet,
- R31: Wasserstoff oder 1-4C-Alkyl bedeutet,
oder worin
- R3 und R31: gemeinsam eine 1-4C-Alkylengruppe bedeuten,
- R4: Wasserstoff oder 1-4C-Alkyl bedeutet,
- R5: Wasserstoff bedeutet,
- R51: Wasserstoff bedeutet,
oder worin
- R5 und R51: gemeinsam eine zusätzliche Bindung darstellen,
- R6: einen durch R61 substituierten Pyridylrest darstellt oder einen durch R7 und R8 substituierten Phenylrest darstellt, wobei
- R61: Wasserstoff, Hydroxy, Halogen, 1-4C-Alkoxy, 1-4C-Alkyl, Carboxyl, Trifluprmethyl, 1-4C-Alkoxycarbonyl oder ganz oder teilweise durch Fluor substituiertes 1-4C-Alkoxy bedeutet,
- R7: Hydroxy, Halogen, Cyano, 1-4C-Alkyl, 1-4C-Alkoxy, 1-4C-Alkylcarbonyloxy, Trifluormethyl, Phenyl, Phenyl-1-4C-alkyl, Nitro, Amino, ganz oder teilweise durch Fluor substituiertes 1-4C-Alkoxy, SO₂-R70 oder N(R71)R72 bedeutet, wobei
R70 1-4C-Alkyl,
R71 Wasserstoff, 1-4C-Alkyl, SO₂-R9 oder SO₂-R10 und
R72 1-4C-Alkyl, 1-4C-Alkylcarbonyl oder SO₂-R10 bedeutet,
- R8: Wasserstoff, Hydroxy, Halogen, 1-4C-Alkoxy oder 1-4C-Alkyl bedeutet
und wobei
- R9 und R10: unabhängig voneinander 1-4C-Alkyl, Phenyl, Phenyl-1-4C-alkyl oder durch einen oder mehrere gleiche oder verschiedene Substituenten substituiertes Phenyl bedeuten, wobei die Substituenten ausgewählt sind aus der Gruppe Nitro, 1-4C-Alkyl, Halogen, 1-4C-Alkylcarbonylamino, 1-4C-Alkoxy, ganz oder teilweise durch Fluor substituiertes 1-4C-Alkoxy, Cyano, Phenyl, Naphthyl, Trifluormethyl oder 1-4C-Alkoxycarbonyl,
sowie die Salze dieser Verbindungen.

1-4C-Alkoxy steht für Reste, die neben dem Sauerstoffatom einen geradkettigen oder verzweigten Alkylrest mit 1 bis 4 Kohlenstoffatomen enthalten. Beispielsweise seien genannt der Butoxy-, iso-Butoxy-, sec.-Butoxy-, tert.-Butoxy-, Propoxy-, Isopropoxy- und bevorzugt der Ethoxy- und Methoxyrest.

3-7C-Cycloalkoxy steht beispielsweise für Cyclopropyloxy, Cyclobutyloxy, Cyclopentyloxy, Cyclohexyloxy und Cycloheptyloxy, wovon Cyclopropyloxy, Cyclobutyloxy und Cyclopentyloxy bevorzugt sind.

3-7C-Cycloalkylmethoxy steht beispielsweise für Cyclopropylmethoxy, Cyclobutylmethoxy, Gyclopentylmethoxy, Cyclohexylmethoxy und Cycloheptylmethoxy, wovon Cyclopropylmethoxy, Cyclobutylmethoxy und Cyclopentylmethoxy bevorzugt sind.

Als ganz oder teilweise durch Fluor substituiertes 1-4C-Alkoxy seien beispielsweise der 1,2,2-Trifluorethoxy-, der 2,2,3,3,3-Pentafluorpropoxy-, der Perfiuorethoxy-, der 1,1,2,2-Tetrafluorethoxy-, der Trifluormethoxy-, insbesondere der 2,2,2-Trifluorethoxy- und bevorzugt der Difluormethoxyrest genannt.

1-4C-Alkyl steht für einen geradkettigen oder verzweigten Alkylrest mit 1 bis 4 Kohlenstoffatomen. Beispielsweise seien genannt der Butyl-, iso-Butyl-, sec.-Butyl-, tert.-Butyl-, Propyl-, Isopropyl- und bevorzugt der Ethyl- und Methylrest.

1-2C-Alkylendioxy steht beispielsweise für den Methylendioxy-(-O-CH₂-O-) und den Ethylendioxyrest (-O-CH₂-CH₂-O-).

Haben R3 und R31 gemeinsam die Bedeutung 1-4C-Alkylen, so sind die Positionen 1 und 4 in Verbindungen der Formel I durch eine 1-4C-Alkylenbrücke miteinander verknüpft, wobei 1-4C-Alkylen für geradkettige oder verzweigte Alkylenreste mit 1 bis 4 Kohlenstoffatomen steht. Beispielsweise seien die Reste Methylen (-CH₂-), Ethylen (-CH₂CH₂), Trimethylen (-CH₂CH₂-CH₂-), 1,2-Dimethylethylen [-CH(CH₃)-CH(CH₃)-] und Isopropyliden [-C(CH₃)₂-] genannt.

Halogen im Sinne der vorliegenden Erfindung ist Brom, Chlor und Fluor.

1-4C-Alkoxycarbonyl enthält neben der Carbonylgruppe einen der oben genannten 1-4C-Alkoxyreste. Beispielsweise genannt seien der Methoxycarbonyl- und der Ethoxycarbonylrest.

Wenn R5 und R51 gemeinsam eine zusätzliche Bindung bedeuten, dann sind die Kohlenstoffatome in den Positionen 2 und 3 in Verbindungen der Formel I über eine Doppelbindung miteinander verknüpft.

1-4C-Alkylcarbonyloxyreste enthalten neben dem Carbonyloxyrest einen der vorstehend genannten 1-4C-Alkylreste. Beispielsweise sei der Acetyloxyrest (CH₃CO-O-) genannt.

Phenyl-1-4C-alkyl steht für einen der obengenannten 1-4C-Alkylreste, der durch einen Phenylrest substituiert ist. Beispielsweise genannt seien der Benzyl- und der Phenethylrest.

1-4C-Alkylcarbonyl steht für einen Rest, der neben der Carbonylgruppe einen der vorstehend genannten 1-4C-Alkylreste enthält. Beispielsweise genannt sei der Acetylrest.

1-4C-Alkylcarbonylamino steht für einen Aminorest, der durch einen der vorstehend genannten 1-4C-Alkylcarbonylreste substituiert ist. Beispielsweise genannt sei der Acetylaminorest (CH₃CO-NH-).

Als beispielhaft durch R61 substituierte Pyridylreste seien die Reste Pyridyl-4, Pyridyl-3, 2-Chlorpyridyl-4, 2-Hydroxypyridyl-4, 2-Methoxypyridyl-4, 2-Brompyridyl-4, 2-Methylpyridyl-4, 3-Brompyridyl-4, 2-Chlorpyridyl-5, 2-Hydroxypyridyl-5, 2-Methoxypyridyl-5, 2-Methylpyridyl-5, 2-Brompyridyl-5, 3-Brompyridyl-5, 2-Methylpyridyl-3, 2-Chlorpyridyl-3, 4-Methylpyridyl-3, 3-Methoxypyridyl-5, 2-(2,2,2-Trifluorethoxy)pyridyl-3, 3-Methylpyridyl-4, 2-Methoxypyridyl-3, 2-Fluorpyridyl-3, 2-Trifluormethylpyridyl-3, 2-Methoxycarbonylpyridyl-3, 4-Trifluormethylpyridyl-3, 4-Methoxycarbonylpyridyl-3 oder 2-(2,2,2-Trifluorethoxy)pyridyl-5 genannt.

Als beispielhafte, durch R7 und R8 substituierte Phenylreste seien die Reste 4-Acetamidophenyl, 3-Acetamidophenyl, 4-Acetoxyphenyl, 3-Aminophenyl, 4-Aminophenyl, 2-Bromphenyl, 4-Bromphenyl, 2-Chlorphenyl, 3-Chlorphenyl, 4-Chlorphenyl, 3-Bromphenyl, 2,3-Dichlorphenyl, 2,4-Dichlorphenyl, 2-Chlor-4-nitrophenyl, 4-Diethylamino-2-methylphenyl, 4-Methoxyphenyl, 3-Methoxyphenyl, 2-Chlor-5-nitrophenyl, 4-Chlor-3-nitrophenyl, 2,6-Dichlorphenyl, 3,5-Dichlorphenyl. 2,5-Dichlorphenyl, 2.6-Dibromphenyl, 2-Cyanphenyl, 3-Cyanphenyl, 4-Cyanphenyl, 4-Diethylaminophenyl, 4-Dimethylaminophenyl, 2-Fluorphenyl, 4-Fluorphenyl, 3-Fluorphenyl, 2,4-Difluorphenyl, 2,6-Difluorphenyl, 2-Chlor-6-fluorphenyl, 2-Fluor-5-nitrophenyl, 2-Hydroxyphenyl, 3-Hydroxyphenyl, 3,4-Dichlorphenyl, 4-Hydroxyphenyl, 4-Hydroxy-3-methoxyphenyl, 2-Hydroxy-4-methoxyphenyl, 2,4-Dihydroxyphenyl, 2-Melhoxyphenyl, 2,3-Dimethoxyphenyl, 3,4-Dimethoxyphenyl, 2,4-Dimethoxyphenyl, 2-Dimethylaminophenyl, 2-Methylphenyl, 3-Methylphenyl, 4-Methylphenyl, 2-Chlor-6-methylphenyl, 4-Methyl-3-nitrophenyl, 2,4-Dimethylphenyl, 2,6-Dimethylphenyl, 2,3-Dimethylphenyl, 2-Nitrophenyl, 3-Nitrophenyl, 4-Nitrophenyl, 2,4-Dinitrophenyl, 3,4-Dinitrophenyl, 3,5-Dinitrophenyl, 2,6-Dinitrophenyl, 4-Ethoxyphenyl, 2-Trifluormethylphenyl, 4-Trifluormethylphenyl, 3-Trifluormethylphenyl, 4-(p-Nitrophenylsulfonamido)-phenyl, 3-(p-Toluolsulfonamido)phenyl, 4-(p-Toluolsulfonamido)phenyl, 4-(4-Ethylphenylsulfonamido)-phenyl, 4-Bis(p-toluoisulfonyl)aminophenyl, 4-Bis(p-nitrophenylsulfonyl)amlnophenyl, 3-(p-Nitrophenylsulfonamido)phenyl, (N-Acetyl-4-p-toluolsulfonamido)phenyl, 4-(Benzylsulfonamido)phenyl, 3-(Benzylsulfonamido)phenyl, 4-(Methylsulfonamido)phenyl, 3-(Methylsulfonamido)phenyl, 4-(N-Methyl-methylsulfonamido)phenyl, 3-(N-Methyl-methylsulfonamido)phenyl, 4-(3,4-Dichlorphenylsulfonamido)phenyl, 3-(3,4-Dichlorphenylsulfonamido)phenyl, 4-(3-Nitrophenylsulfonamido)phenyl, 3-(3-Nitrophenylsulfonamido)phenyl, 4-(4-Bromphenylsulfonamido)phenyl, 3-(4-Bromphenylsulfonamido)phenyl, 4-(3-Bromphenylsulfonamido)phenyl, 3-(3-Bromphenylsulfonamido)phenyl, 4-(3-Fluorphenylsulfonamido)phenyl, 3-(3-Fluorphenylsulfonamido)phenyl, 4-(4-Fluorphenylsulfonamido)phenyl, 3-(4-Fluorphenylsulfonamido)phenyl, 4-(4-Chlorphenylsulfonamido)phenyl, 3-(4-Chlorphenylsulfonamido)phenyl, 4-(3-Chlorphenylsulfonamido)phenyl, 3-(3-Chlorphenylsulfonamido)phenyl, 4-(4-Acetylaminophenylsulfonamido)-phenyl, 3-(4-Acetylaminophenylsulfonamido)phenyl, 4-(4-Methoxyphenylsulfonamido)phenyl, 3-(4-Methoxyphenylsulfonamido)phenyl, 4-(3-Trifluormethylphenylsulfonamido)phenyl, 3-(3-Trifluormethylphenylsulfonamido)phenyl, 4-(4-Trifluormethylphenylsulfonamido)phenyl, 3-(4-Trifluormethylphenylsulfonamido)phenyl, 4-(4-Trifluormethoxyphenylsulfonamido)phenyl, 3-(4-Trifluormethoxyphenylsulfonamido)phenyl, 4-(3-Methylphenylsulfonamido)phenyl, 3-(3-Methylphenylsulfonamido)phenyl, 4-(3,4-Dimethoxyphenylsulfonamido)phenyl, 3-(3,4-Dimethoxyphenylsulfonamido)phenyl, 4-(4-Cyanophenylsulfonamido)phenyl, 3-(4-Cyanophenylsulfonamido)phenyl, 4-(3-Cyanophenylsulfonamido)phenyl, 3-(3-Cyanophenylsulfonamido)phenyl, 4-(3-Chlor-4-methylphenylsulfonamido)phenyl, 3-(3-Chlor-4-methylphenylsulfonamido)phenyl, 4-(4-Biphenylsulfonamido)phenyl, 3-(4-Biphenylsulfonamido)phenyl, 4-(4-Isopropylphenylsulfonamido)phenyl, 3-(4-Isopropylphenylsulfonamido)phenyl, 4-(Naphth-1-ylsulfonamido)phenyl, 3-(Naphth-1-ylsulfonamido)phenyl, 4-(Naphth-2-ylsulfonamido)phenyl, 3-(Naphth-2-ylsulfonamido)phenyl, 4-Benzylphenyl, 4-Biphenyl, 4-Trifluormethoxyphenyl, 3-Trifluormethoxyphenyl, 2-Trifluormethoxyphenyl, 4-Methansulfonylphenyl, 3-Methansulfonylphenyl, 2-Methansulfonylphenyl, 4-(4-Methoxycarbonylphenylsulfonamido)phenyl oder (N-Methyl-4-p-toluolsulfonamido)phenyl genannt.

Hervorzuhebende Verbindungen der Formel I sind solche, worin
- R1: Hydroxy, 1-4C-Alkoxy, 3-7C-Cycloalkoxy, 3-7C-Cycloalkylmethoxy oder ganz oder teilweise durch Fluor substituiertes 1-4C-Alkoxy bedeutet,
- R2: Hydroxy, 1-4C-Alkoxy, 3-7C-Cycloalkoxy, 3-7C-Cycloalkylmethoxy oder ganz oder teilweise durch Fluor substituiertes 1-4C-Alkoxy bedeutet,
oder worin
- R1 und R2: gemeinsam eine 1-2C-Alkylendioxygruppe bedeuten,
- R3: Wasserstoff oder 1-4C-Alkyl bedeutet,
- R31: Wasserstoff oder 1-4C-Alkyl bedeutet,
oder worin
- R3 und R31: gemeinsam eine 1-4C-Alkylengruppe bedeuten,
- R4: Wasserstoff oder 1-4C-Alkyl bedeutet,
- R5: Wasserstoff bedeutet,
- R51: Wasserstoff bedeutet,
oder worin
- R5 und R51: gemeinsam eine zusätzliche Bindung darstellen,
- R6: einen durch R61 substituierten Pyridylrest darstellt oder einen durch R7 und R8 substituierten Phenylrest darstellt, wobei
- R61: Wasserstoff, Hydroxy, Halogen, 1-4C-Alkoxy, 1-4C-Alkyl, Carboxyl, Trifluormethyl, 1-4C-Alkoxycarbonyl oder ganz oder teilweise durch Fluor substituiertes 1-4C-Alkoxy bedeutet,
- R7: Hydroxy, Halogen, Cyano, 1-4C-Alkyl, 1-4C-Alkoxy, 1-4C-Alkylcarbonyloxy, Trifluormethyl, Phenyl, Phenyl-1-4C-alkyl, Nitro, Amino oder N(R71)R72 bedeutet, wobei
R71 Wasserstoff, 1-4C-Alkyl, SO₂-R9 oder SO₂-R10 und
R72 1-4C-Alkyl, 1-4C-Alkylcarbonyl oder SO₂-R10 bedeutet,
- R8: Wasserstoff, Hydroxy, Halogen, 1-4C-Alkoxy oder 1-4C-Alkyl bedeutet
und wobei
- R9 und R10: unabhängig voneinander 1-4C-Alkyl, Phenyl, Phenyl-1-4C-alkyl oder durch einen oder mehrere gleiche oder verschiedene Substituenten substituiertes Phenyl bedeuten, wobei die Substituenten ausgewählt sind aus der Gruppe Nitro, 1-4C-Alkyl, Halogen, 1-4C-Alkylcarbonylamino, 1-4C-Alkoxy, ganz oder teilweise durch Fluor substituiertes 1-4C-Alkoxy, Cyano, Phenyl, Naphthyl oder Trifluorrnethyl,
sowie die Salze dieser Verbindungen.

Eine Ausgestaltung [Ausgestaltung a)] der erfindungsgemäßen Verbindungen sind Verbindungen der Formel I, worin
- R1: Hydroxy, 1-4C-Alkoxy, 3-7C-Cycloalkoxy, 3-7C-Cydoalkylmethoxy oder ganz oder teilweise durch Fluor substituiertes 1-4C-Alkoxy bedeutet,
- R2: Hydroxy, 1-4C-Alkoxy, 3-7C-Cycloalkoxy, 3-7C-Cydoalkylmethoxy oder ganz oder teilweise durch Fluor substituiertes 1-4C-Alkoxy bedeutet,
oder worin
- R1 und R2: gemeinsam eine 1-2C-Alkylendioxygruppe bedeuten,
- R3: Wasserstoff oder 1-4C-Alkyl bedeutet,
- R31: Wasserstoff oder 1-4C-Alkyl bedeutet,
oder worin
- R3 und R31: gemeinsam eine 1-4C-Alkylengruppe bedeuten,
- R4: Wasserstoff oder 1-4C-Alkyl bedeutet,
- R5: Wasserstoff bedeutet,
- R51: Wasserstoff bedeutet,
oder worin
- R5 und R51: gemeinsam eine zusätzliche Bindung darstellen,
- R6: einen durch R61 substituierten Pyridylrest darstellt, wobei
- R61: Wasserstoff, Hydroxy, Halogen, 1-4C-Alkoxy, 1-4C-Alkyl, Carboxyl, Trifluormethyl, 1-4C-Alkoxycarbonyl oder ganz oder teilweise durch Fluor substituiertes 1-4C-Alkoxy bedeutet,
sowie die Salze dieser Verbindungen.

Hervorzuhebende Verbindungen der Ausgestaltung a) sind Verbindungen der Formel I, worin
- R1: 1-4C-Alkoxy, 3-7C-Cycloalkoxy, 3-7C-Cycloalkylmethoxy oder ganz oder teilweise durch Fluor substituiertes 1-2C-Alkoxy bedeutet,
- R2: 1-4C-Alkoxy, 3-7C-Cycloalkoxy, 3-7C-Cycloalkylmethoxy oder ganz oder teilweise durch Fluor substituiertes 1-2C-Alkoxy bedeutet,
- R3: Wasserstoff bedeutet,
- R31: Wasserstoff bedeutet,
oder worin
- R3 und R31: gemeinsam eine 1-2C-Alkylengruppe bedeuten,
- R4: Wasserstoff oder 1-4C-Alkyl bedeutet,
- R5: Wasserstoff bedeutet,
- R51: Wasserstoff bedeutet,
oder worin
- R5 und R51: gemeinsam eine zusätzliche Bindung darstellen,
- R6: einen durch R61 substituierten Pyridylrest darstellt, wobei
- R61: Wasserstoff, Hydroxy, Halogen oder 1-4C-Alkoxy bedeutet,
sowie die Salze dieser Verbindungen.

Besonders hervorzuhebende Verbindungen der Ausgestaltung a) sind Verbindungen der Formel I, worin
- R1: 1-4C-Alkoxy, 3-7C-Cycloalkoxy oder ganz oder teilweise durch Fluor substituiertes 1-2C-Alkoxy bedeutet,
- R2: 1-4C-Alkoxy, 3-7C-Cycloalkoxy oder ganz oder teilweise durch Fluor substituiertes 1-2C-Alkoxy bedeutet,
- R3: Wasserstoff bedeutet,
- R31: Wasserstoff bedeutet,
oder worin
- R3 und R31: gemeinsam eine 1-2C-Alkylengruppe bedeuten,
- R4: Wasserstoff oder 1-4C-Alkyl bedeutet,
- R5: Wasserstoff bedeutet,
- R51: Wasserstoff bedeutet,
oder worin
- R5 und R51: gemeinsam eine zusätzliche Bindung darstellen,
- R6: einen durch R61 substituierten Pyridylrest darstellt, wobei
- R61: Wasserstoff, Hydroxy, Halogen oder 1-4C-Alkoxy bedeutet,
sowie die Salze dieser Verbindungen.

Bevorzugte Verbindungen der Ausgestaltung a) sind Verbindungen der Formel I, worin
- R1: 1-4C-Alkoxy bedeutet,
- R2: 1-4C-Alkoxy bedeutet,
- R3: Wasserstoff bedeutet,
- R31: Wasserstoff bedeutet,
- R4: Wasserstoff bedeutet,
- R5: Wasserstoff bedeutet,
- R51: Wasserstoff bedeutet,
- R6: einen durch R61 substituierten Pyridylrest darstellt, wobei
- R61: Wasserstoff, Hydroxy oder Halogen bedeutet,
sowie die Salze dieser Verbindungen.

Eine andere Ausgestaltung [Ausgestaltung b)] der erfindungsgemäßen Verbindungen sind solche Verbindungen der Formel I, worin
- R1: Hydroxy, 1-4C-Alkoxy, 3-7C-Cycloalkoxy, 3-7C-Cycloalkylmethoxy oder ganz oder teilweise durch Fluor substituiertes 1-4C-Alkoxy bedeutet,
- R2: Hydroxy, 1-4C-Alkoxy, 3-7C-Cydoalkoxy, 3-7C-Cycloalkylmethoxy oder ganz oder teilweise durch Fluor substituiertes 1-4C-Alkoxy bedeutet,
oder worin
- R1 und R2: gemeinsam eine 1-2C-Alkylendioxygruppe bedeuten,
- R3: Wasserstoff oder 1-4C-Alkyl bedeutet,
- R31: Wasserstoff oder 1-4C-Alkyl bedeutet,
oder worin
- R3 und R31: gemeinsam eine 1-4C-Alkylengruppe bedeuten,
- R4: Wasserstoff oder 1-4C-Alkyl bedeutet,
- R5: Wasserstoff bedeutet,
- R51: Wasserstoff bedeutet,
oder worin
- R5 und R51: gemeinsam eine zusätzliche Bindung darstellen,
- R6: einen durch R7 und R8 substituierten Phenylrest darstellt, wobei
- R7: Hydroxy, Halogen, Cyano, 1-4C-Alkyl, 1-4C-Alkoxy, 1-4C-Alkylcarbonyloxy, Trifluormethyl, Phenyl, Phenyl-1-4C-alkyl, Nitro, Amino oder N(R71)R72 bedeutet, wobei
R71 Wasserstoff, 1-4C-Alkyl, SO₂-R₉ oder SO₂-R10 und
R72 1-4C-Alkyl, 1-4C-Alkylcarbonyl oder SO₂-R10 bedeutet,
- R8: Wasserstoff, Hydroxy, Halogen, 1-4C-Alkoxy oder 1-4C-Alkyl bedeutet
und wobei
- R9 und R10: unabhängig voneinander 1-4C-Alkyl, Phenyl, Phenyl-1-4C-alkyl oder durch einen oder mehrere gleiche oder verschiedene Substituenten substituiertes Phenyl bedeuten, wobei die Substituenten ausgewählt sind aus der Gruppe Nitro, 1-4C-Alkyl, Halogen, 1-4C-Alkylcarbonylamino, 1-4C-Alkoxy, ganz oder teilweise durch Fluor substituiertes 1-4C-Alkoxy, Cyano, Phenyl, Naphthyl oder Trifluormethyl,
sowie die Salze dieser Verbindungen.

Hervorzuhebende Verbindungen der Ausgestaltung b) sind Verbindungen der Formel I, worin
- R1: 1-4C-Alkoxy, 3-7C-Cycloalkoxy, 3-7C-Cydoalkylmethoxy oder ganz oder teilweise durch Fluor substituiertes 1-2C-Alkoxy bedeutet,
- R2: 1-4C-Alkoxy, 3-7C-Cycloalkoxy, 3-7C-Cycloalkylmethoxy oder ganz oder teilweise durch Fluor substituiertes 1-2C-Alkoxy bedeutet,
- R3: Wasserstoff bedeutet,
- R31: Wasserstoff bedeutet,
oder worin
- R3 und R31: gemeinsam eine 1-4C-Alkylengruppe bedeuten,
- R4: Wasserstoff oder 1-4C-Alkyl bedeutet,
- R5: Wasserstoff bedeutet,
- R51: Wasserstoff bedeutet,
oder worin
- R5 und R51: gemeinsam eine zusätzliche Bindung darstellen,
- R6: einen durch R7 und R8 substituierten Phenylrest darstellt, wobei
- R7: Hydroxy, Halogen, 1-4C-Alkyl, 1-4C-Alkoxy, 1-4C-Alkylcarbonyloxy, Trifluormethyl, Phenyl, Phenyl-1-4C-alkyl, Nitro, Amino oder N(R71)R72 bedeutet, wobei
R71 Wasserstoff, 1-4C-Alkyl, SO₂-R9 oder SO₂-R10 und
R72 1-4C-Alkyl, 1-4C-Alkylcarbonyl oder SO₂-R10 bedeutet,
- R8: Wasserstoff, Hydroxy, Halogen, 1-4C-Alkoxy oder 1-4C-Alkyl bedeutet
und wobei
- R9 und R10: unabhängig voneinander 1-4C-Alkyl, Phenyl oder durch einen Substituenten substituiertes Phenyl bedeuten, wobei der Substituent ausgewählt ist aus der Gruppe Nitro, 1-4C-Alkyl, Halogen, oder Trifluormethyl,
sowie die Salze dieser Verbindungen.

Besonders hervorzuhebende Verbindungen der Ausgestaltung b) sind Verbindungen der Formel I, worin
- R1: 1-4C-Alkoxy, 3-7C-Cycloalkoxy oder ganz oder teilweise durch Fluor substituiertes 1-2C-Alkoxy bedeutet,
- R2: 1-4C-Alkoxy, 3-7C-Cycloalkoxy oder ganz oder teilweise durch Fluor substituiertes 1-2C-Alkoxy bedeutet,
- R3: Wasserstoff bedeutet,
- R31: Wasserstoff bedeutet,
oder worin
- R3 und R31: gemeinsam eine 1-4C-Alkylengruppe bedeuten,
- R4: Wasserstoff oder 1-4C-Alkyl bedeutet,
- R5: Wasserstoff bedeutet,
- R51: Wasserstoff bedeutet,
oder worin
- R5 und R51: gemeinsam eine zusätzliche Bindung darstellen,
- R6: einen durch R7 und R8 substituierten Phenylrest darstellt, wobei
- R7: Hydroxy, Halogen, 1-4C-Alkoxy, 1-4C-Alkylcarbonyloxy, Phenyl, Phenyl-1-4C-alkyl, Nitro, Amino oder N(R71)R72 bedeutet, wobei
R71 Wasserstoff, SO₂-R9 oder SO₂-R10 und
R72 1-4C-Alkylcarbonyl oder SO₂-R10 bedeutet,
- R8: Wasserstoff, Halogen oder 1-4C-Alkoxy bedeutet
und wobei
- R9 und R10: unabhängig voneinander Phenyl oder durch einen Substituenten substituiertes Phenyl bedeuten, wobei der Substituent ausgewählt ist aus der Gruppe Nitro, 1-4C-Alkyl, Halogen oder Trifluormethyl,
sowie die Salze dieser Verbindungen.

Bevorzugte Verbindungen der Ausgestaltung b) sind Verbindungen der Formel I, worin
- R1: 1-4C-Alkoxy oder 3-7C-Cycloalkoxy bedeutet,
- R2: 1-4C-Alkoxy oder 3-7C-Cycloalkoxy bedeutet,
- R3: Wasserstoff bedeutet,
- R31: Wasserstoff bedeutet.
oder worin
- R3 und R31: gemeinsam eine 1-2C-Alkylengruppe bedeuten,
- R4: Wasserstoff oder 1-4C-Alkyl bedeutet,
- R5: Wasserstoff bedeutet,
- R51: Wasserstoff bedeutet,
oder worin
- R5 und R51: gemeinsam eine zusätzliche Bindung darstellen,
- R6: einen durch R7 und R8 substituierten Phenylrest darstellt, wobei
- R7: Hydroxy, Halogen, 1-4C-Alkoxy, 1-4C-Alkylcarbonyloxy, Phenyl, Phenyl-1-4C-alkyl, Nitro, Amino oder N(R71)R72 bedeutet, wobei
R71 Wasserstoff oder SO₂-R10 und
R72 14C-Alkylcarbonyl oder SO₂-R10 bedeutet,
- R8: Wasserstoff, Halogen oder 1-4C-Alkoxy bedeutet
und wobei
- R10: durch einen Substituenten substituiertes Phenyl bedeutet, wobei der Substituent ausgewählt ist aus der Gruppe Nitro oder 1-4C-Alkyl,
sowie die Salze dieser Verbindungen.

Eine Ausgestaltung [Ausgestaltung c)] der erfindungsgemäßen Verbindungen sind solche Verbindungen der Formel I, worin
- R1: Hydroxy, 1-4C-Alkoxy, 3-7C-Cycloalkoxy, 3-7C-Cycloalkylmethoxy oder ganz oder teilweise durch Fluor substituiertes 1-4C-Alkoxy bedeutet,
- R2: Hydroxy, 1-4C-Alkoxy, 3-7C-Cycloalkoxy, 3-7C-Cycloalkylmethoxy oder ganz oder teilweise durch Fluor substituiertes 1-4C-Alkoxy bedeutet,
oder worin
- R1 und R2: gemeinsam eine 1-2C-Alkylendioxygruppe bedeuten,
- R3: Wasserstoff oder 1-4C-Alkyl bedeutet,
- R31: Wasserstoff oder 1-4C-Alkyl bedeutet,
oder worin
- R3 und R31: gemeinsam eine 1-4C-Alkylengruppe bedeuten,
- R4: Wasserstoff oder 1-4C-Alkyl bedeutet,
- R5: Wasserstoff bedeutet,
- R51: Wasserstoff bedeutet,
oder worin
- R5 und R51: gemeinsam eine zusätzliche Bindung darstellen,
- R6: einen durch R7 und R8 substituierten Phenylrest darstellt, wobei
- R7: Hydroxy, Halogen, Cyano, 1-4C-Alkyl, 1-4C-Alkoxy, 1-4C-Alkylcarbonyloxy, Trifluormethyl, Phenyl, Phenyl-1-4C-alkyl, Nitro, Amino, ganz oder teilweise durch Fluor substituiertes 1-4C-Alkoxy, SO₂-R70 oder N(R71)R72 bedeutet, wobei
R70 1-4C-Alkyl,
R71 Wasserstoff, 1-4C-Alkyl, SO₂-R9 oder SO₂-R10 und
R72 1-4C-Alkyl, 1-4C-Alkylcarbonyl oder SO₂-R10 bedeutet,
- R8: Wasserstoff, Hydroxy, Halogen, 1-4C-Alkoxy oder 1-4C-Alkyl bedeutet
und wobei
- R9 und R10: unabhängig voneinander 1-4C-Alkyl, Phenyl, Phenyl-1-4C-alkyl oder durch einen oder mehrere gleiche oder verschiedene Substituenten substituiertes Phenyl bedeuten, wobei die Substituenten ausgewählt sind aus der Gruppe Nitro, 1-4C-Alkyl, Halogen, 1-4C-Alkylcarbonylamino, 1-4C-Alkoxy, ganz oder teilweise durch Fluor substituiertes 1-4C-Alkoxy, Cyano, Phenyl, Naphthyl, Trifluormethyl oder 1-4C-Alkoxycarbonyl,
sowie die Salze dieser Verbindungen.

Hervorzuhebende Verbindungen der Ausgestaltung c) sind solche Verbindungen der Formel I, worin
- R1: 1-4C-Alkoxy, 3-7C-Cycloalkoxy, 3-7C-Cycloalkylmethoxy oder ganz oder teilweise durch Fluor substituiertes 1-2C-Alkoxy bedeutet,
- R2: 1-4C-Alkoxy, 3-7C-Cycloalkoxy, 3-7C-Cycloalkylmethoxy oder ganz oder teilweise durch Fluor substituiertes 1-2C-Alkoxy bedeutet,
- R3: Wasserstoff bedeutet,
- R31: Wasserstoff bedeutet,
oder worin
- R3 und R31: gemeinsam eine 1-4C-Alkylengruppe bedeuten,
- R4: Wasserstoff oder 1-4C-Alkyl bedeutet,
- R5: Wasserstoff bedeutet,
- R51: Wasserstoff bedeutet,
oder worin
- R5 und R51: gemeinsam eine zusätzliche Bindung darstellen,
- R6: einen durch R7 und R8 substituierten Phenylrest darstellt, wobei
- R7: Hydroxy, Halogen, Cyano, 1-4C-Alkyl, 1-4C-Alkoxy, 1-4C-Alkylcarbonyloxy, Trifluormethyl, Phenyl, Phenyl-1-4C-alkyl, Nitro, Amino, ganz oder teilweise durch Fluor substituiertes 1-4C-Alkoxy, SO₂-R70 oder N(R71)R72 bedeutet, wobei
R70 1-4C-Alkyl,
R71 Wasserstoff, 1-4C-Alkyl, SO₂-R9 oder SO₂-R10 und
R72 1-4C-Alkyl, 1-4C-Alkylcarbonyl oder SO₂-R10 bedeutet,
- R8: Wasserstoff, Hydroxy, Halogen, 1-4C-Alkoxy oder 1-4C-Alkyl bedeutet
und wobei
- R9 und R10: unabhängig voneinander 1-4C-Alkyl, Phenyl oder durch einen Substituenten substituiertes Phenyl bedeuten, wobei der Substituent ausgewählt ist aus der Gruppe Nitro, 1-4C-Alkyl, Halogen, Trifluormethyl oder 1-4C-Alkoxycarbonyl,
sowie die Salze dieser Verbindungen.

Besonders hervorzuhebende Verbindungen der Ausgestaltung c) sind solche Verbindungen der Formel l, worin
- R1: 1-4C-Alkoxy, 3-7C-Cycloalkoxy oder ganz oder teilweise durch Fluor substituiertes 1-2C-Alkoxy bedeutet,
- R2: 1-4C-Alkoxy, 3-7C-Cycloalkoxy oder ganz oder teilweise durch Fluor substituiertes 1-2C-Alkoxy bedeutet,
- R3: Wasserstoff bedeutet,
- R31: Wasserstoff bedeutet,
oder worin
- R3 und R31: gemeinsam eine 1-2C-Alkylengruppe bedeuten,
- R4: Wasserstoff oder 1-4C-Alkyl bedeutet,
- R5: Wasserstoff bedeutet,
- R51: Wasserstoff bedeutet,
oder worin
- R5 und R51: gemeinsam eine zusätzliche Bindung darstellen,
- R6: einen durch R7 und R8 substituierten Phenylrest darstellt, wobei
- R7: Hydroxy, Halogen, Cyano, 1-4C-Alkoxy, 1-4C-Alkylcarbonyloxy, Trifluormethyl, Phenyl, Phenyl-1-4C-alkyl, Nitro, Amino, ganz oder teilweise durch Fluor substituiertes 1-4C-Alkoxy, SO₂-R70 oder N(R71)R72 bedeutet, wobei
R70 1-4C-Alkyl,
R71 Wasserstoff, 1-4C-Alkyl, SO₂-R9 oder SO₂-R10 und
R72 1-4C-Alkylcarbonyl oder SO₂-R10 bedeutet,
- R8: Wasserstoff, Halogen oder 1-4C-Alkoxy bedeutet
und wobei
- R9 und R10: unabhängig voneinander Phenyl oder durch einen Substituenten substituiertes Phenyl bedeuten, wobei der Substituent ausgewählt ist aus der Gruppe Nitro, 1-4C-Alkyl, Halogen, Trifluormethyl oder 1-4C-Alkoxycarbonyl,
sowie die Salze dieser Verbindungen.

Bevorzugte Verbindungen der Ausgestaltung c) sind solche Verbindungen der Formel I, worin
- R1: 1-4C-Alkoxy oder 3-7C-Cycloalkoxy bedeutet,
- R2: 1-4C-Alkoxy oder 3-7C-Cycloalkoxy bedeutet,
- R3: Wasserstoff bedeutet,
- R31: Wasserstoff bedeutet,
oder worin
- R3 und R31: gemeinsam eine 1-2C-Alkylengruppe bedeuten,
- R4: Wasserstoff oder 1-4C-Alkyl bedeutet,
- R5: Wasserstoff bedeutet,
- R51: Wasserstoff bedeutet,
oder worin
- R5 und R51: gemeinsam eine zusätzliche Bindung darstellen,
- R6: einen durch R7 und R8 substituierten Phenylrest darstellt, wobei
- R7: Hydroxy, Halogen, Cyano, 1-4C-Alkoxy, 1-4C-Alkylcarbonyloxy, Trifluormethyl, Phenyl, Phenyl-1-4C-alkyl, Nitro, Amino, ganz oder teilweise durch Fluor substituiertes 1-4C-Alkoxy, SO₂-R70 oder N(R71)R72 bedeutet, wobei
R70 1-4C-Alkyl,
R71 Wasserstoff, 1-4C-Alkyl oder SO₂-R10 und
R72 1-4C-Alkylcarbonyl oder SO₂-R10 bedeutet,
- R8: Wasserstoff, Halogen oder 1-4C-Alkoxy bedeutet
und wobei
- R10: durch einen Substituenten substituiertes Phenyl bedeutet, wobei der Substituent ausgewählt ist aus der Gruppe Nitro, 1-4C-Alkyl oder 1-4C-Alkoxycarbonyl,
sowie die Salze dieser Verbindungen.

Besonders bevorzugte Verbindungen der Formel I sind solche, worin
- R1: 1-4C-Alkoxy oder 3-7C-Cycloalkoxy bedeutet,
- R2: 1-4C-Alkoxy oder 3-7C-Cycloalkoxy bedeutet,
- R3: Wasserstoff bedeutet,
- R31: Wasserstoff bedeutet,
- R4: Wasserstoff oder 1-4C-Alkyl bedeutet,
- R5: Wasserstoff bedeutet,
- R51: Wasserstoff bedeutet,
oder worin
- R5 und R51: gemeinsam eine zusätzliche Bindung darstellen,
- R6: einen durch R7 und R8 substituierten Phenylrest darstellt, wobei
- R7: N(R71)R72 bedeutet, wobei
R71 Wasserstoff, SO₂-R9 oder SO₂-R10 und
R72 1-4C-Alkyl, 1-4C-Alkylcarbonyl oder SO₂-R10 bedeutet,
- R8: Wasserstoff bedeutet
und wobei
- R9 und R10: unabhängig voneinander durch einen Substituenten substituiertes Phenyl bedeuten, wobei der Substituent ausgewählt ist aus der Gruppe Nitro, 1-4C-Alkyl oder 1-4C-Alkoxycarbonyl,
sowie die Salze dieser Verbindungen.

Als Salze kommen für Verbindungen der Formel I - je nach Substitution - alle Säureadditionssalze oder alle Salze mit Basen in Betracht. Besonders erwähnt seien die pharmakologisch verträglichen Salze der in der Galenik üblicherweise verwendeten anorganischen und organischen Säuren und Basen. Als solche eignen sich einerseits wasserlösliche und wasserunlösliche Säureadditionssalze mit Säuren wie beispielsweise Salzsäure, Bromwasserstoffsäure, Phosphorsäure, Salpetersäure, Schwefelsäure, Essigsäure, Zitronensäure, D-Gluconsäure, Benzoesäure, 2-(4-Hydroxybenzoyl)-benzoesäure, Buttersäure, Sulfosalicylsäure, Maleinsäure, Laurinsäure, Äpfelsäure, Fumarsäure, Bernsteinsäure.

Oxalsäure, Weinsäure, Embonsäure, Stearinsäure, Toluolsulfonsäure, Methansulfonsäure oder 3-Hydroxy-2-naphthoesäure, wobei die Säuren bei der Salzherstellung - je nachdem, ob es sich um eine ein- oder mehrbasige Säure handelt und je nachdem, welches Salz gewünscht wird - im äquimolaren oder einem davon abweichenden Mengenverhältnis eingesetzt werden.

Andererseits kommen auch Salze mit Basen in Betracht. Als Beispiele für Salze mit Basen seien Alkali- (Lithium-, Natrium-, Kalium-) oder Calcium-, Aluminium-, Magnesium-, Titan-, Ammonium-, Meglumin- oder Guanidiniumsalze erwähnt, wobei auch hier bei der Salzherstellung die Basen im äquimolaren oder einem davon abweichenden Mengenverhältnis eingesetzt werden.

Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt.

Bei den Verbindungen der Formel I handelt es sich um chirale Verbindungen mit Chiralitätszentren in den Positionen 4a und 10b und je nach Bedeutung der Substituenten R3, R31, R4, R5 und R51 weiteren Chiralitätszentren in den Positionen 1,2,3 und 4. Die Erfindung umfaßt daher alle denkbaren reinen Diastereomeren und reinen Enantiomeren als auch deren Gemische in jedem Mischungsverhältnis, einschließlich der Racemate. Bevorzugt sind die Verbindungen der Formel I, in denen die Wasserstoffatome in den Positionen 4a und 10b cis-ständig zueinander sind. Insbesondere bevorzugt sind dabei die reinen cis-Diastereomeren und die reinen cis-Enantiomeren als auch deren Gemische in jedem Mischungsverhältnis und einschließlich der Racemate.

Die Enantiomeren können in an sich bekannter Weise (beispielsweise durch Herstellung und Trennung entsprechender diastereoisomerer Verbindungen) separiert werden. Vorzugsweise erfolgt eine Enantiomerentrennung auf Stufe der Ausgangsverbindungen der Formel III (siehe beigefügtes Formelblatt) beispielsweise über Salzbildung der racemischen Verbindungen der Formel III mit optisch aktiven Carbonsäuren. Alternativ können enantiomerenreine Ausgangsverbindungen der Formel III auch über asymmetrische Synthesen dargestellt werden.

Weiterer Gegenstand der Erfindung ist ein Verfahren zur Herstellung der Verbindungen der Formel I, worin R1, R2, R3, R31, R4, R5, R51 und R6 die oben angegebenen Bedeutungen besitzen, und ihrer Salze. Das Verfahren ist dadurch gekennzeichnet, daß man
a) entsprechende Verbindungen der Formel II (siehe beigefügtes Pormelblatt), in denen R1, R2, R3, R31, R4, R5, R51 und R6 die oben angegebenen Bedeutungen besitzen, cyclokondensiert, oder daß man
b) zur Herstellung von Verbindungen der Formel I, worin R1, R2, R3, R31, R4, R5 und R51 die oben angegebenen Bedeutungen besitzen und R6 durch R61 substituiertes Pyridyl darstellt, wobei R61 Hydroxy bedeutet, entsprechende Verbindungen der Formel I, worin R1, R2, R3, R31, R4, R5 und R51 die oben angegebenen Bedeutungen besitzen und R6 durch R61 substituiertes Pyridyl darstellt, wobei R61 Halogen bedeutet, hydrolysiert oder daß man
c) zur Herstellung von Verbindungen der Formel I, worin R1, R2, R3, R31, R4, R5 und R51 die oben genannten Bedeutungen haben und R6 einen durch R7 und R8 substituierten Phenylrest darstellt, wobei R7 Amino bedeutet, entsprechende Verbindungen der Formel I, worin R7 Nitro bedeutet, reduziert, oder daß man
d) zur Herstellung von Verbindungen der Formel I, worin R1, R2, R3, R31, R4, R5 und R51 die oben genannten Bedeutungen haben und R6 einen durch R7 und R8 substituierten Phenylrest darstellt, wobei R7 Hydroxy bedeutet, entsprechende Verbindungen der Formel I, worin R7 1-4C-Alkylcarbonyloxy bedeutet, hydrolysiert, oder daß man
e) Verbindungen der Formel I, worin R1, R2, R3, R31, R4, R5 und R51 die oben genannten Bedeutungen haben und R6 einen durch R7 und R8 substituierten Phenylrest darstellt, wobei R7 Amino bedeutet, mit einem geeignet aktivierten 1-4C-Alkylcarbonylderivat N-acyliert, oder daß man
f) Verbindungen der Formel I, worin R1, R2, R3, R31, R4, R5 und R51 die oben genannten Bedeutungen haben und R6 einen durch R7 und R8 substituierten Phenylrest darstellt, wobei R7 Amino bedeutet, mit einer Sulfonsäureverbindung der Formel X-SO₂-R10, worin R10 die oben genannte Bedeutung besitzt und X eine geeignete Abgangsgruppe darstellt, umsetzt, oder daß man
g) Verbindungen der Formel I, worin R1, R2, R3, R31, R4, R5 und R51 die oben genannten Bedeutungen haben und R6 einen durch R7 und R8 substituierten Phenylrest darstellt, wobei R7 die Gruppe N(H)R72 bedeutet, wobei R72 die Bedeutung 1-4C-Alkylcarbonyl oder SO₂-R10 hat, mit einem geeignet aktivierten 1-4C-Alkylcarbonylderivat N-acyliert, mit einem geeignet aktivierten 1-4C-Alkylderivat N-alkyliert oder mit einer Sulfonsäureverbindung der Formel X-SO₂-R9 oder X-SO₂-R10, worin R9 und R10 die oben genannten Bedeutungen besitzen und X eine geeignete Abgangsgruppe darstellt, umsetzt, und daß man
gewünschtenfalls anschließend nach a), b), c), d), e), f) oder g) erhaltene Verbindungen der Formel I in ihre Salze überführt, oder daß man gewünschtenfalls anschließend nach a), b), c), d), e), f) oder g) erhaltene Salze der Verbindungen der Formel I in die freien Verbindungen überführt.

Die Cyclokondensation nach Verfahrensvariante a) erfolgt auf eine dem Fachmann an sich bekannte Weise gemäß Bischler-Napieralski (z.B. so, wie in J.Chem.Soc., **1956,** 4280-4282 beschrieben) in Gegenwart eines geeigneten Kondensationsmittels, wie beispielsweise Polyphosphorsäure, Phosphorpentachlorid, Phosphorpentoxid oder bevorzugt Phosphoroxytrichlorid, in einem geeigneten inerten Lösungsmittel, z.B. in einem chlorierten Kohlenwasserstoff wie Chloroform, oder in einem cyclischen Kohlenwasserstoff wie Toluol oder Xylol, oder einem sonstigen inerten Lösungsmittel wie Acetonitril, oder ohne weiteres Lösungsmittel unter Verwendung eines Überschusses an Kondensationsmittel, vorzugsweise bei erhöhter Temperatur, insbesondere bei der Siedetemperatur des verwendeten Lösungs- bzw. Kondensationsmittels.

Die Hydrolyse von Verbindungen der Formel I, worin R61 Halogen, insbesondere Brom oder Chlor bedeutet, nach Variante b) erfolgt ebenfalls auf eine dem Fachmann bekannte Weise (z.B. so, wie in Helv.Chim.Acta **1942**, 25, 1485 beschrieben), in einem geeigneten Lösungsmittel und in Gegenwart oder Abwesenheit von Wasser, durch Umsetzung mit einer oder mehreren geeigneten Basen, bei Reaktionstemperaturen zwischen 0°C und der Siedetemperatur des verwendeten Lösungsmittels.

Geeignete Basen sind beispielsweise Metallcarbonate wie Kalium- und Natriumcarbonat und Metallhydroxide wie Kalium- und Natriumhydroxid, wobei bei Reaktionsführung unter Ausschluß von Wasser vorzugsweise eine Kombination aus Metallcarbonat und Metallhydroxid verwendet wird. Gewünschtenfalls kann die Reaktionsführung bei Verwendung eines organischen Lösungsmittels durch Zusatz eines Komplexierungsreagenzes verbessert werden (z.B. so, wie in Tetrahedron **1987**, 43, 2557 beschrieben).

Die Reduktion der Nitroverbindungen analog Variante c) erfolgt zweckmäßigerweise durch katalytische Hydrierung, beispielsweise mit Raney-Nickel und molekularem Wasserstoff oder einer anderen Wasserstoffquelle wie Hydrazin, mit unedlen Metallen wie Zinn, Zink und Eisen (vorzugsweise in saurer Lösung), auf elektrolytische Weise oder mit sonstigen geeigneten Reduktionsmitteln, in einem geeigneten Lösungsmittel, z.B. in Wasser oder einem Alkohol wie Methanol oder Ethanol und gewünschtenfalls in Gegenwart einer Säure wie beispielsweise Salzsäure.

Insbesondere bevorzugt ist die Reduktion mit Eisen und Salzsäure, z.B. so wie in den Beispielen beschrieben.

Die Hydrolyse nach Variante d) erfolgt ebenfalls auf eine dem Fachmann bekannte Weise, bevorzugt unter basischen Bedingungen, beispielsweise mit einer Base wie Kaliumhydroxid in einem geeigneten Lösungsmittel, z.B. einem Alkohol wie Methanol und in Gegenwart oder Abwesenheit von Wasser.

Die N-Acylierung nach Variante e) erfolgt in einer Weise, wie sie für die Herstellung von Amiden bekannt ist. Geeignet aktivierte 1-4C-Alkylcarbonylderivate sind beispielsweise entsprechende Säuren, Ester, Azide und insbesondere Anhydride und Halogenide (bevorzugt Chloride und Bromide).

Die Reaktion kann gewünschtenfalls in Gegenwart einer geeigneten Base, z.B. eines Alkalimetallcarbonates wie Kaliumcarbonat, eines Alkalimetallhydroxids wie Natriumhydroxid oder einer Stickstoffbase wie Pyridin, Triethylamin oder Ethyldiisopropylamin und oder unter Einsetzung eines Überschusses an Amin der Formel I erfolgen. Alternativ kann die Umsetzung auch ohne Base durchgeführt werden, wobei - je nach Art der Ausgangsverbindungen - gegebenenfalls zuerst die Säureadditionssalze in besonders reiner Form abgetrennt werden können.

Als geeignete Lösungsmittel für die Acylierung seien beispielsweise genannt Lösungsmittel wie Dimethylsulfoxid, Aceton, Tetrahydrofuran, Dimethylformamid oder Acetonitril, oder auch chlorierte Kohlenwasserstoffe wie Methylenchlorid. Gewünschtenfalls kann die Reaktion auch ohne zusätzliches Lösungsmittel unter Verwendung eines Überschusses an Acylierungsmittel und oder Base als Lösungsmittel erfolgen.

Die Darstellung von erfindungsgemäßen Sulfonamiden der Formel I nach Variante f) erfolgt in einer Weise wie sie für die Synthese von Sulfonamiden bekannt ist, beispielsweise analog der N-Acylierung nach Variante e). Bevorzugte Sulfonsäurederivate der Formel X-SO₂-R10, die dabei Verwendung finden sind solche, worin X eine geeignete Abgangsgruppe wie beispielsweise Halogen, insbesondere Chlor, bedeutet.

Je nachdem, ob Verbindungen der Formel I, worin R7 N(H)SO₂-R10 oder N(SO₂-R10)₂ bedeutet, das gewünschte Produkt sind, wird das Sulfonsäurederivat der Formel X-SO₂-R10 im äquimolaren oder einem davon abweichenden Mengenverhältnis eingesetzt.

Die Umsetzung gemäß Variante g) kann ebenfalls auf eine dem Fachmann bekannte Weise erfolgen, beispielsweise analog Variante e) oder f). Die Verbindungen der Formel I, worin R7 die Gruppe N(H)R72 bedeutet, wobei R72 die Bedeutung 1-4C-Alkylcarbonyl oder SO₂-R10 hat, können dabei als solche oder bevorzugt in Form ihrer Salze mit Basen, z.B. in Form der Alkalisalze (insbesondere als Natriumsalz) eingesetzt werden. Zweckmäßigerweise wird das Salz unmittelbar vor der Umsetzung durch Deprotonierung mit einer geeigneten Base, beispielsweise mit einem Metallhydrid wie Natriumhydrid, in einem vorzugsweise aprotischen dipolaren Lösungsmittel wie Dimethylformamid oder Tetrahydrofuran aus den entsprechenden freien Verbindungen der Formel I erzeugt.

Die Isolierung und Reinigung der erfindungsgemäßen Substanzen erfolgt in an sich bekannter Weise z.B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Salze erhält man durch Auflösen der freien Verbindung in einem geeigneten Lösungsmittel, z.B. in einem chlorierten Kohlenwasserstoff, wie Methylenchlorid oder Chloroform, oder einem niedermolekularen aliphatischen Alkohol (Ethanol, Isopropanol), das die gewünschte Säure bzw. Base enthält, oder dem die gewünschte Säure bzw. Base anschließend zugegeben wird. Die Salze werden durch Filtrieren, Umfällen, Ausfällen mit einem Nichtlösungsmittel für das Anlagerungssalz oder durch Verdampfen des Lösungsmittels gewonnen. Erhaltene Salze können durch Alkalisierung bzw. durch Ansäuem in die freien Verbindungen umgewandelt werden, welche wiederum in Salze übergeführt werden können. Auf diese Weise lassen sich pharmakologisch nicht verträgliche Salze in pharmakologisch verträgliche Salze umwandeln.

Verbindungen der Formel II (siehe beigefügtes Formelblatt), worin R1, R2, R3, R31, R4, R5, R51 und R6 die oben angegebenen Bedeutungen haben, sind aus den entsprechenden Verbindungen der Formel III (siehe beigefügtes Formelblatt), worin R1, R2, R3, R31, R4, R5 und R51 die oben angegebenen Bedeutungen haben, durch Umsetzung mit Verbindungen der Formel R6-CO-X, worin R6 die oben angegebene Bedeutung hat und X eine geeignete Abgangsgruppe, vorzugsweise ein Chloratom darstellt, zugänglich. Beispielsweise wird die Acylierung bzw. Benzoylierung wie in den nachfolgenden Beispielen oder wie in J.Chem.Soc.(C), **1971**, 1805-1808 beschrieben durchgeführt.

Verbindungen der Formel R6-CO-X und Verbindungen der Formel III sind entweder bekannt oder können auf bekannte Weise hergestellt werden.

Die Verbindungen der Formel III lassen sich z.B. aus Verbindungen der Formel IV (siehe beigefügtes Formelblatt), worin R1, R2, R3, R31, R4, R5 und R51 die oben genannten Bedeutungen haben, durch Reduktion der Nitrogruppe darstellen.

Die Reduktion erfolgt auf eine dem Fachmann bekannte Weise, beispielsweise so wie in J.Org.Chem. **1962**, 27, 4426 oder wie in den folgenden Beispielen beschrieben. Vorzugsweise erfolgt die Reduktion durch katalytische Hydrierung, z.B. in Gegenwart von Raney-Nickel, in einem niederen Alkohol wie Methanol oder Ethanol bei Raumtemperatur und unter Normal- oder erhöhtem Druck. Gewünschtenfalls kann dem Lösungsmittel eine katalytische Menge einer Säure, wie beispielsweise Salzsäure zugesetzt werden.

Die Verbindungen der Formel IV (siehe beigefügtes Formelblatt), worin R1, R2, R3, R31 und R4 die oben angegebenen Bedeutungen haben und R5 und R51 Wasserstoff bedeuten, sind entweder bekannt oder können aus entsprechenden Verbindungen der Formel IV, worin R5 und R51 gemeinsam eine zusätzliche Bindung bedeuten, hergestellt werden. Die Reaktion kann auf eine dem Fachmann bekannte Weise erfolgen, vorzugsweise durch Hydrierung in Gegenwart eines Katalysators, wie beispielsweise Palladium auf Aktivkohle, z.B. so wie in J.Chem.Soc.(C), **197**1, 1805-1808 oder wie in den folgenden Beispielen beschrieben.

Die Verbindungen der Formel IV, worin R5 und R51 gemeinsam eine zusätzliche Bindung bedeuten, sind entweder bekannt oder können durch Umsetzung von Verbindungen der Formel V (siehe beigefügtes Formelblatt), worin R1 und R2 die oben genannten Bedeutungen haben, mit Verbindungen der Formel VI (siehe beigefügtes Formelblatt), worin R3, R31 und R4 die oben genannten Bedeutungen besitzen, erhalten werden.

Die Cycloaddition erfolgt dabei auf eine dem Fachmann bekannte Weise gemäß Diels-Alder, z.B. so wie in J.Amer.Chem.Soc. **1957**, 79, 6559 oder in J.Org.Chem. **1952**, 17, 581 oder wie in den folgenden Beispielen beschrieben.

Bei der Cycloaddition erhaltene Verbindungen der Formel IV, worin der Phenylring und die Nitrogruppe trans-ständig zueinander sind, können in einer dem Fachmann bekannten Weise in die entsprechenden cis-Verbindungen übergeführt werden z.B. so wie in J.Amer.Chem.Soc. **1957**, 79, 6559 oder wie in den nachfolgenden Beispielen beschrieben.

Die Verbindungen der Formel VI und V sind entweder bekannt oder können auf bekannte Weise hergestellt werden. Die Verbindungen der Formel V können beispielsweise auf eine dem Fachmann bekannte Weise aus entsprechenden Verbindungen der Formel VII, so wie z.B. in J.Chem.Soc. **1951**, 2524 oder in J.Org.Chem. **1944,** 9, 170 oder wie in den folgenden Beispielen beschrieben, hergestellt werden.

Die Verbindungen der Formel VII (siehe beigefügtes Formelblatt), worin R1 und R2 die oben angegebenen Bedeutungen haben, sind entweder bekannt oder können auf eine dem Fachmann bekannte Weise, so wie z.B. in Ber.Dtsch.Chem. Ges. **1925**, 58, 203 oder wie in den folgenden Beispielen beschrieben, hergestellt werden.

Die nachfolgenden Beispiele dienen der näheren Erläuterung der Erfindung ohne sie einzuschränken. Ebenso können weitere Verbindungen der Formeln I, deren Herstellung nicht explizit beschrieben ist, in analoger oder in einer dem Fachmann an sich vertrauten Weise unter Anwendung üblicher Verfahrenstechniken hergestellt werden.

In den Beispielen steht Schmp. für Schmelzpunkt, d.Th. für "der Theorie", Sdp. für Siedepunkt, h für Stunde(n), RT für Raumtemperatur, SF für Summenformel, MG für Molgewicht, Ber. für Berechnet, Gef. für Gefunden. Die in den Beispielen genannten Verbindungen und ihre Salze sind bevorzugter Gegenstand der Erfindung.

### Beispiele

### Endprodukte

### 1. (+/-)-cis-8,9-Dimethoxy-6-(4-p-toluolsulfonamidophenyl)-1,2,3,4,4a,10b-hexahydrophenanthridin

3,5 g (+/-)-cis-N-[2-(3,4-Dimethoxyphenyl)cyclohexyl]-4-p-toluolsulfonamidobenzamid werden in 100 ml Acetonitril und 1,0 ml Phosphoroxychlorid gelöst und 8 h bei 50°C gerührt. Das Reaktionsgemisch wird in 100 ml gesättigte Natriumhydrogencarbonatlösung gegeben und mit Essigsäureethylester extrahiert. Die organische Phase wird mit Natriumhydrogencarbonatlösung und Wasser gewaschen, mit Natriumsulfat getrocknet und eingeengt. Der Rückstand wird aus Ethanol umkristallisiert. Man erhält 1,8 g (53,2 % d.Th.) der Titelverbindung mit Schmp.: 122°C (Zersetzung).
SF: C₂₈ H₃₀ N₂ O₄ S; MG: 490.63

| | | | | | |
|---|---|---|---|---|---|
| Elementaranalyse | Ber. | C 68.54 | H 6.16 | N 5.71 | S 6.53 |
| | Gef. | C 68.35 | H 6.24 | N 5.55 | S 6.47 |

Ausgehend von den nachfolgend beschriebenen Ausgangsverbindungen erhält man entsprechend der Arbeitsweise nach Beispiel 1:

### 2. (+/-)-cis-8,9-Diethoxy-6-(4-p-toluolsulfonamidophenyl)-1,2,3,4,4a,10b-hexahydrophenanthridin

Schmp.: 110-115°C, Ausbeute 42,4 % d.Th.
SF: C₃₀ H₃₄ N₂ O₄ S; MG: 518.68

| | | | | | |
|---|---|---|---|---|---|
| Elementaranalyse | Ber. | C 68.99 | H 6.64 | N 5.36 | S 6.14 |
| | Gef. | C 69.04 | H 6.58 | N 5.28 | S 6.12 |

### 3. (+/-)-cis-9-Cyclopentyloxy-8-methoxy-6-(4-p-toluolsulfonamidophenyl)-1,2,3,4,4a,10bhexahydrophenanthridin

Schmp.: 175°C, Ausbeute 62,5 % d.Th.
SF: C₃₂ H₃₆ N₂ O₄ S; MG: 544.72

| | | | | | |
|---|---|---|---|---|---|
| Elementaranalyse | Ber. | C 70.56 | H 6.66 | N 5.14 | S 5.89 |
| | Gef. | C 70.09 | H 6.72 | N 5.05 | S 5.88 |

### 4. (+/-)-cis-8-Cyclopentyloxy-9-methoxy-6-(4-p-toluolsulfonamidophenyl)-1,2,3,4,4a,10bhexahydrophenanthridin

Schmp.: 193-196°C, Ausbeute 38,9 % d.Th.
SF: C₃₂ H₃₆ N₂ O₄ S; MG: 544.72

| | | | | | |
|---|---|---|---|---|---|
| Elementaranalyse: | Ber.: | C 70.56 | H 6.66 | N 5.14 | S 5.89 |
| | Gef. | C 70.39 | H 6.77 | N 5.10 | S 5.73 |

### 5. (+/-)-cis-8,9-Dimethoxy-6-(4-nitrophenyl)-1,2,3,4,4a,10b-hexahydrophenanthridin

Schmp.: 182°C, Ausbeute 75,2 % d.Th.
SF: C₂₁ H₂₂ N₂ O₄; MG: 366.42

| | | | | |
|---|---|---|---|---|
| Elementaranalyse | Ber. | C 68.84 | H 6.05 | N 7.65 |
| | Gef. | C 68.88 | H 6.10 | N 7.57 |

### 6. (+/-)-cis-8,9-Dimethoxy-6-(2,6-dichlorphenyl)-1,2,3,4,4a,10b-hexahydrophenanthridin

Schmp.: 174-175°C, Ausbeute 44,0 % d.Th.
SF: C₂₁ H₂₁ Cl₂ N O₂ ; MG: 390.31

| | | | | | |
|---|---|---|---|---|---|
| Elementaranalyse | Ber. | C 64.62 | H 5.42 | Cl 18.17 | N 3.59 |
| | Gef. | C 64.45 | H 5.40 | Cl 17.89 | N 3.62 |

### 7. (+/-)-cis-8,9-Dimethoxy-6-(3,4-dimethoxyphenyl)-1,2,3,4,4a,10b-hexahydrophenanthridin

Schmp.: 160-162°C, Ausbeute 61,3 % d.Th.
SF: C₂₃ H₂₇ N O₄ ; MG: 381.48

| | | | | |
|---|---|---|---|---|
| Elementaranalyse | Ber. | C 72.42 | H 7.13 | N 3.67 |
| | Gef. | C 72.38 | H 7.00 | N 3.53 |

### 8. (+/-)-cis-8,9-Dimethoxy-2,3-dimethyl-6-(4-p-toluolsulfonamidophenyl)-1,4,4a,10b-tetrahydrophenanthridin

Schmp. ab 128°C (Zersetzung).
SF: C₃₀ H₃₂ N₂ O₄ S; MG: 516.66

| | | | | |
|---|---|---|---|---|
| Elementaranalyse (x 0,5 H₂O) | Ber. | C 68.55 | H 6.33 | N 5.33 |
| | Gef. | C 68.91 | H 6.29 | N 5.31 |

### 9. (+/-)-trans-8,9-Dimethoxy-6-(4-p-toluolsulfonamidophenyl)-1,2,3,4,4a,10b-hexahydrophenanthridin-hydrochtorid

Schmp.: 219-222°C, Ausbeute 30,2 % d.Th.
SF: C₂₈ H₃₀ N₂ O₄ S x H Cl x H₂ O ; MG: 545.1

| | | | | | | |
|---|---|---|---|---|---|---|
| Elementaranalyse | Ber. | C 61.70 | H 6.10 | Cl 6.50 | N 5.14 | S 5.88 |
| | Gef. | C 61.94 | H 6.00 | Cl 6.79 | N 5.15 | S 5.83 |

### 10. (+/-)-trans-8,9-Dimethoxy-6-(4-p-toluolsulfonamidophenyl)-1,4,4a,10b-tetrahydrophenanthridin-hydrochlorid

Schmp.: 189-192°C, Ausbeute 40,6 % d.Th.
SF: C₂₈ H₂₈ N₂ O₄ S x H Cl X H₂ O; MG: 543.09

| | | | | | | |
|---|---|---|---|---|---|---|
| Elementaranalyse | Ber. | C 61.93 | H 5.75 | Cl 6.53 | N 5.16 | S 5.90 |
| | Gef. | C 61.44 | H 5.51 | Cl 6.78 | N 5.04 | S 5.83 |

### 11. (+/-)-trans-8,9-Dimethoxy-2,3-dimethyl-6-(4-p-toluolsulfonamidophenyl)-1,4,4a,10b-tetrahydrophenanthridin

Schmp.: 200-203.5°C, Ausbeute 54,2 % d.Th.
SF: C₃₀ H₃₂ N₂ O₄ S; MG: 516.66

| | | | | | |
|---|---|---|---|---|---|
| Elementaranalyse | Ber. | C 69.74 | H 6.24 | N 5.42 | S 6.21 |
| | Gef. | C 69.67 | H 6.37 | N 5.37 | S 6.02 |

### 12. (+/-)-trans-8,9-Dimethoxy-1,4-ethano-6-(4-p-toluolsulfonamidophenyl)-1,2,3,4,4a,10bhexahydrophenanthridin

Schmp.: >220°C (Zersetzung).
SF: C₃₀ H₃₂ N₂ O₄ S; MG: 516.66

### 13. (+/-)-cis-6-(4-Acetoxyphenyl)-8,9-diethoxy-1,2,3,4,4a,10b-hexahydrophenanthridin

Schmp.: 99-101°C, Ausbeute 31,3 % d.Th.
SF: C₂₅ H₂₉ N O₄; MG: 407.51

| | | | | |
|---|---|---|---|---|
| Elementaranalyse | Ber. | C 73.69 | H 7.17 | N 3.44 |
| | Gef. | C 73.47 | H 7.15 | N 3.47 |

### 14. (+/-)-cis-6-(4-Benzylphenyl)-8,9-diethoxy-1,2,3,4,4a,10b-hexahydrophenanthridin

Schmp.: 135-137°C, Ausbeute 65,1 % d.Th.
SF: C₃₀ H₃₃ N O₂; MG: 439.6

| | | | | |
|---|---|---|---|---|
| Elementaranalyse | Ber. | C 81.97 | H 7.56 | N 3.18 |
| | Gef. | C 81.93 | H 7.54 | N 3.43 |

### 15. (+/-)-cis-6-Biphenyl-8,9-diethoxy-1,2,3,4,4a,10b-hexahydrophenanthridin

Schmp.: 156-158°C, Ausbeute 38,6 % d.Th.
SF: C₂₉ H₃₁ N O₂; MG: 425.58

| | | | | |
|---|---|---|---|---|
| Elementaranalyse | Ber. | C 81.85 | H 7.34 | N 3.29 |
| | Gef. | C 81.69 | H 7.34 | N 3.17 |

### 16. (+/-)-cis-8,9-Diethoxy-6-(4-fluorphenyl)-1,2,3,4,4a,10b-hexahvdrophenanthridin

Schmp.: 92-93°C, Ausbeute 20,3 % d.Th.
SF: C₂₃H₂₆FNO₂; MG: 367.47

| | | | | |
|---|---|---|---|---|
| Elementaranalyse | Ber. | C 75.18 | H 7.13 | N 3.81 |
| | Gef. | C 75.21 | H 7.19 | N 3.74 |

### 17. (+/-)-cis-8,9-Diethoxy-7-(4-trifluormethylphenyl)-1,2,3,4,4a,10b-hexahydrophenanthridin

Öl, Ausbeute 27,5 % d.Th.
SF: C₂₄H₂₆F₃NO₂; MG: 417.48

| | | | | |
|---|---|---|---|---|
| Elementaranalyse | Ber. | C 69.05 | H 6.28 | N 3.36 |
| | Gef. | C 68.70 | H 6.31 | N 3.09 |

### 18. (+/-)-cis-8,9-Diethoxy-6-(4-cyanophenyl)-1,2,3,4,4a,10b-hexahydrophenanthridin

Schmp.: 125-127°C, Ausbeute 59,9 % d.Th.
SF: C₂₄H₂₆N₂O₂; MG: 374.49

| | | | | |
|---|---|---|---|---|
| Elementaranalyse | Ber. | C 76.98 | H 7.00 | N 7.48 |
| | Gef. | C 76.92 | H 7.15 | N 7.37 |

### 19. (+/-)-cis-8,9-Diethoxy-6-(4-trifluormethoxyphenyl)-1,2,3,4,4a,10b-hexahydrophenanthridin

Schmp.: 98-100°C, Ausbeute 23,4 % d.Th.
SF: C₂₄H₂₆F₃NO₃; MG: 433.48

| | | | | |
|---|---|---|---|---|
| Elementaranalyse | Ber. | C 66.50 | H 6.05 | N 3.23 |
| | Gef. | C 66.44 | H 6.05 | N 3.18 |

### 20. (+/-)-cis-9-Ethoxy-8-methoxy-6-(4-p-toluolsulfonamidophenyl)-1,2,3,4,4a,10b-hexahydrophenanthridin

Schmp.: 95°C (Zers.), Ausbeute 51,0 % d.Th.
SF: C₂₉H₃₂N₂O₄S; MG: 504.65

| | | | | |
|---|---|---|---|---|
| Elementaranalyse: | Ber. | C 69.02 | H 6.39 | N 5.55 |
| | Gef. | C 69.19 | H 6.68 | N 5.44 |

### 21. (+/-)-cis-9-Ethoxy-8-methoxy-6-(4-nitrophenyl)-1,2,3,4,4a,10b-hexahydrophenanthridin

Schmp.: 122-124°C, Ausbeute 60,5 % d.Th.
SF: C₂₂H₂₄N₂O₄; MG: 380.45

| | | | | |
|---|---|---|---|---|
| Elementaranalyse. | Ber. | C 69.46 | H 6.36 | N 7.36 |
| | Gef. | C 69.21 | H 6.23 | N 7.24 |

### 22. (+/-)-cis-9-Ethoxy-8-methoxy-6-(4-methansulfonylphenyl)-1,2,3,4,4a,10b-hexahydrophenanthridin

Schmp.: 196-197°C, Ausbeute 67,2 % d.Th.
SF: C₂₃H₂₇NO₄; MG: 413.54

| | | | | |
|---|---|---|---|---|
| Elementaranalyse | Ber. | C 66.80 | H 6.58 | N 3.39 |
| | Gef. | C 66.85 | H 6.62 | N 3.45 |

### 23. (+/-)-cis-9-Ethoxy-8-methoxy-6-[4-(4-methoxycarbonylphenyl)sulfonamidophenyl]-1,2,3,4,4a,10b-hexahvdrophenanthridin Hydrochlorid

Schmp.: Zers. ab 210°C, Ausbeute 55,6 % d.Th.
SF: C₃₀H₃₂N₂O₆S x HCI; MG: 585.12

| | | | | | | |
|---|---|---|---|---|---|---|
| Elementaranalyse x 0.5 H₂O | Ber. | C 60.65 | H 5.77 | N 4.72 | Cl 5.97 | S 5.40 |
| | Gef. | C 60.51 | H 5.68 | N 4.73 | Cl 5.89 | S 5.93 |

### 24. (+/-)-cis-8,9-Diethoxy-6-(pyrid-4-yl)-1,2,3,4,4a,10b-hexahydrophenanthridin-dihydrochlorid

3,2 g (+/-)-cis-N-[2-(3,4-Diethoxyphenyl)cyclohexyl]4-isonicotinsäureamid werden in 50 ml Acetonitril und 3,0 ml Phosphoroxychlorid gelöst und 8 h bei 50°C gerührt. Das Reaktionsgemisch wird in 100 ml gesättigte Natriumhydrogencarbonatlösung gegeben und mit Essigsäureethylester extrahiert. Die organische Phase wird mit Natriumhydrogencarbonatlösung und Wasser gewaschen, mit Natriumsulfat getrocknet und eingeengt. Der Rückstand wird über Kieselgel mit einer Mischung aus Toluol/Dioxan/-Petroläther/Triethylamin im Verhältnis 6:2:1:0.5 chromatographiert. Die Produktfraktionen werden eingeengt, der Rückstand in 30 ml Ethanol gelöst, mit 7 ml mit Chlorwasserstoffgas gesättigtem Diethylether versetzt und in 400 ml Diethylether getropft. Der Niederschlag wird abgesaugt, mit Diethylether nachgewaschen und getrocknet. Man erhält 1,6 g (43,4 % d.Th.) der Titelverbindung als Dihydrochlorid mit Schmp.: 223°C (Zers.).
SF: C₂₂ H₂₆ N₂ O₂ x 2 HCl; MG: 423.39

| | | | | | |
|---|---|---|---|---|---|
| Elementaranalyse | Ber. | C 57.52 | H 5.42 | Cl 18.17 | N 3.59 |
| | Gef. | C 57.09 | H 5.40 | Cl 17.89 | N 3.62 |

Ausgehend von den nachfolgend beschriebenen Ausgangsverbindungen erhält man entsprechend der Arbeitsweise nach Beispiel 24:

### 25. (+/-)-cis-8,9-Diethoxy-6-(pyrid-3-yl)-1,2,3,4,4a,10b-hexahydrophenanthridin-hydrochlorid

Schmp.: 233°C, Ausbeute 37,8% d.Th.
SF: C₂₂H₂₆N₂O₂ x HCl; MG: 386.93

| | | | | | |
|---|---|---|---|---|---|
| Elementaranalyse | Ber. | C 68.29 | H 7.03 | Cl 9.16 | N 7.24 |
| | Gef. | C 68.43 | H 7.09 | Cl 9.29 | N 7.30 |

### 26. (+/-)-cis-8,9-Diethoxy-6-(2-chiorpyrid-5-yl)-1,2,3,4,4a,10b-hexahydrophenanthridin

Die Titelverbindung erhält man durch Einengen der Produktfraktionen nach Chromatographie analog der Arbeitsweise nach Beispiel 24:
Schmp.: 123-126°C, Ausbeute 75,7 % d.Th.
SF: C₂₂ H₂₅ Cl N₂ O₂: MG: 384.91

| | | | | | |
|---|---|---|---|---|---|
| Elementaranalyse | Ber. | C 68.01 | H 6.59 | Cl 9.13 | N 7.21 |
| | Gef. | C 68.04 | H 6.45 | Cl 9.26 | N 7.16 |

### 27. (+/-)-cis-8,9-Diethoxy-6-(2-hydroxypyrid-5-yl)-1,2,3,4,4a,10b-hexahydrophenanthridin

850 mg (+/-)-cis-8,9-Diethoxy-6-(2-chlorpyrid-5-yl)-1,2,3,4,4a,10b-hexahydrophenanthridin werden in 60 ml Toluol suspendiert, mit 1,0 g Kaliumcarbonat, 2,0 g Kaliumhydroxid und 0,26 mg Tris [2-(2-methoxyethoxy)ethyl]amin versetzt und über Nacht unter Rückfluß gekocht. Die Suspension wird filtriert, das Lösungsmittel im Vakuum entfemt, der Rückstand in Wasser aufgenommen, mit 0,1 M Salzsäure neutralisiert und mit Essigsäureethylester extrahiert. Die organische Phase wird mit Natriumsulfat getrocknet, das Lösungsmittel im Vakuum entfernt, der dabei ausfallende Niederschlag wird abgesaugt und getrocknet. Schmp.: 211-213°C, Ausbeute 12,3 % d.Th.
SF: C₂₂ H₂₆ N₂ O₃; MG: 366.46

| | | | | |
|---|---|---|---|---|
| Elementaranalyse | Ber. | C 72.11 | H 7.15 | N 7.64 |
| | Gef. | C 72.55 | H 7.10 | N 7.58 |

### 28. (+/-)-cis-8,9-Dimethoxy-6-(4-aminophenyl)-1,2,3,4,4a,10b-hexahydrophenanthridin

8,5g (+/-)-cis-8,9-Dimethoxy-6-(4-nitrophenyl)-1,2,3,4,4a,10b-hexahydrophenanthridin werden in 200 ml Methanol gelöst, mit 5 ml konzentrierter Salzsäure, 20 ml Wasser und 800 mg Eisenpulver versetzt und über Nacht bei RT gerührt. Die Reaktionsmischung wird filtriert, das Filtrat eingeengt, der Rückstand mit Natriumhydrogencarbonatlösung/Essigsäureethylester extrahiert, die organische Phase mit Natriumsulfat getrocknet und eingeengt. Der Rückstand wird über Kieselgel mit einem Gemisch aus Toluol/Dioxan/Triethylamin im Verhältnis 40:20:2 chromatographiert. Nach Einengen der entsprechenden Eluatfraktionen erhält man 5,5 g (70,5 % d.Th.) der Titelverbindung mit Schmp. 159,5-161°C.
SF: C₂₁H₂₄ N₂ O₂; MG: 336.44

Ausgehend von den nachfolgend beschriebenen Ausgangsverbindungen erhält man entsprechend der Arbeitsweise nach Beispiel 28:

### 29. (+/-)-cis-9-Ethoxy-8-methoxy-6-(4-aminophenyl)-1,2,3,4,4a,10b-hexahydrophenanthridin

Schmp.: 183-186°C, Ausbeute 30,8 % d.Th.
SF: C₂₂H₂₆N₂O₂; MG: 350.47

| | | | | |
|---|---|---|---|---|
| Elementaranalyse | Ber. | C 75.40 | H 7.48 | N 7.99 |
| | Gef. | C 75.72 | H 7.53 | N 7.70 |

### 30. (+/-)-cis-8,9-Dimethoxy-6-[4-bis(p-nitrophenylsulfonyl)aminophenyl]-1,2,3,4,4a,10b-hexahydrophenanthridin

Zu einer Lösung von 2,0 g (+/-)-cis-8,9-Dimethoxy-6-(4-aminophenyl)-1,2,3,4,4a,10b-hexahydrophenanthridin in 50 ml Methylenchlorid und 1,0 ml Triethylamin werden 1,45 g 4-Nitrobenzolsulfonsäurechlorid in 20 ml Methylenchlorid getropft. Die Lösung wird über Nacht bei RT gerührt, mit Wasser extrahiert, die organische Phase getrocknet und eingeengt. Der Rückstand wird über Kieselgel mit einem Gemisch aus Petroläther/Essigsäureethylester im Verhältnis 3:2 chromatographiert. Nach Einengen der entsprechenden Eluatfraktionen erhält man die Titelverbindung mit Schmp. 234.5°C.
SF: C₃₃ H₃₀ N₄ O₁₀ S₂; MG: 706.76

| | | | | | |
|---|---|---|---|---|---|
| Elementaranalyse | Ber. | C 56.08 | H 4.28 | N 7.93 | S 9.07 |
| | Gef. | C 55.93 | H 4.29 | N 7.73 | S 8.86 |

### 31. (+/-)-cis-8,9-Dimethoxy-6-(4-acetamidophenyl)-1,2,3,4,4a,10b-hexahydrophenanthridin

1,0 g (+/-)-cis-8,9-Dimethoxy-6-(4-aminophenyl)-1,2,3,4,4a,10b-hexahydrophenanthridin werden in 10 ml Essigsäureanhydrid suspendiert und 1 h bei RT gerührt. Die Lösung wird mit Diethylether versetzt, der Niederschlag wird abgesaugt und mit Natriumhydrogencarbonatlösung/Essigsäureethylester extrahiert. Nach Trocknen und Einengen der organischen Phase wird der Rückstand aus Essigsäureethylester/Methanol umkristallisiert. Man erhält 0,63 g (56,0 % d.Th.) der Titelverbindung mit Schmp. 218°C.
SF: C₂₃ H₂₆ N₂ O₃; MG: 378.48

| | | | | |
|---|---|---|---|---|
| Elementaranalyse | Ber. | C 72.99 | H 6.92 | N 7.40 |
| | Gef. | C 72.93 | H 6.91 | N 7.33 |

Ausgehend von den nachfolgend beschriebenen Ausgangsverbindungen erhält man entsprechend der Arbeitsweise nach Beispiel 31:

### 32. (+/-)-cis-9-Ethoxy-8-methoxy-6-(4-acetamidophenyl)-1,2,3,4,4a,10b-hexahydrophenanthridin

erstarrendes Öl, Ausbeute 69,7 % d.Th.
SF: C₂₄H₂₈N₂O₃; MG 392.50

| | | | | |
|---|---|---|---|---|
| Elementaranalyse | Ber. | C 73.44 | H 7.19 | N 7.14 |
| | Gef. | C 73.01 | H 7.38 | N 6.68 |

### 33. (+/-)-cis-8,9-Dimethoxy-6-[4-bis(p-toluolsulfonyl)aminophenyl]-1,2,3,4,4a,10b-hexahydrophenanthridin

Zu einer Suspension von 200 mg 80 %igem Natriumhydrid in 20 ml Dimethylformamid werden 2,0 g (+/-)-cis-8,9-Dimethoxy-6-(4-p-toluolsulfonamidophenyl)1,2,3,4,4a,10b-hexahydrophenanthridin in 5 ml Dimethylformamid und anschließend 1,0 g p-Toluolsulfonsäurechlorid in 5 ml Dimethylformamid getropft. Nach 2 h Rühren bei RT wird auf Eiswasser gegeben und mit Essigsäureethylester/Diethylether im Verhältnis 1:1 extrahiert. Nach Trocknen und Einengen der organischen Phase wird der Rückstand über Kieselgel mit einer Mischung aus Essigsäureethylester/Petroläther im Verhältnis 4:1 chromatographiert. Nach Einengen der entsprechenden Eluatfraktionen erhält man die Titelverbindung mit Schmp. 120-130°C.
SF: C₃₅ H₃₆ N₂ O₆ S₂; MG: 644.81

| | | | | | |
|---|---|---|---|---|---|
| Elementaranalyse | Ber. | C 64.30 | H 5.70 | N 4.28 | S 9.81 |
| | Gef. | C 64.19 | H 5.71 | N 4.22 | S 9.74 |

### 34. (+/-)-cis-8,9-Dimethoxy-6-[(N-acetyl-4-p-toluolsulfonamido)phenyl]-1,2,3,4,4a,10b-hexahydrophenanthridin

Zu einer Suspension von 100 mg 80 %igem Natriumhydrid in 10 ml Dimethylformamid werden 1,5 g (+/-)-cis-8,9-Dimethoxy-6-(4-p-toluolsulfonamidophenyl)-1,2,3,4,4a,10b-hexahydrophenanthridin in 5 ml Dimethylformamid und anschließend 0,5 ml Acetylchlorid in 5 ml Dimethylformamid getropft. Man läßt über Nacht bei RT rühren, gibt auf Natriumhydrogencarbonatlösung und extrahiert mit Essigsäureethylester. Nach Trocknen und Einengen der organischen Phase wird der Rückstand über Kieselgel mit einer Mischung aus Toluol/Dioxan im Verhältnis 2:1 chromatographiert. Nach Einengen der entsprechenden Eluatfraktionen erhält man die Titelverbindung mit Schmp. 212-216°C.
SF: C₃₀ H₃₂ N₂ O₅ S; MG: 532.66

| | | | | | |
|---|---|---|---|---|---|
| Elementaranalyse: | Ber. | C 67.65 | H 6.06 | N 5.26 | S 6.02 |
| | Gef. | C 67.71 | H 6.03 | N 5.21 | S 5.83 |

Ausgehend von den nachfolgend beschriebenen Ausgangsverbindungen erhält man entsprechend der Arbeitsweise nach Beispiel 34:

### 35. (+/-)-cis-8,9-Diethoxy-6-[(N-acetyl-4-p-toluolsulfonamido]phenyl]-1,2,3,4,4a,10b-hexahydrophenanthridin

erstarrtes Öl, Ausbeute 41,6 % d.Th.
SF: C₃₂ H₃₆N₂ O₅ S; MG: 560.72

| | | | | | |
|---|---|---|---|---|---|
| Elementaranalyse | Ber. | C 68.55 | H 6.74 | N 5.00 | S 5.72 |
| | Gef. | C 68.71 | H 6.49 | N 4.81 | S 5.52 |

### 36. (+/-)-cis-9-Ethoxy-8-methoxy-6-[(N-methyl-4-p-toluolsulfonamido)phenyl]-1,2,3,4,4a,10bhexahydrophenanthridin

60 mg 80 %iges Natriumhydrid werden unter Stickstoff in 10 ml Dimethylformamid suspendiert, mit 500 mg (+/-)-cis-9-Ethoxy-8-methoxy-6-(4-p-toluolsulfonamidophenyl)-1,2,3,4,4a,10b-hexahydrophenanthridin gelöst in 5 ml Dimethylformamid und 70 µl Methyljodid gelöst in 5 ml Dimethylformamid versetzt und über Nacht bei RT gerührt. Nach Hydrolyse mit Wasser wird mit Diethylether extrahiert und anschließend die organische Phase mit Natriumsulfat getrocknet und eingeengt. Der Rückstand wird über Kieselgel mit einem Gemisch aus Petroläther/Essigsäureethylester/Methanol im Verhältnis 6/3/1 chromatographiert. Nach Einengen der entsprechenden Eluatfraktionen erhält man 150 mg (28,9 % d.Th.) der Titelverbindung als erstarrendes Öl.
SF: C₃₀H₃₄N₂O₄S; MG: 518.68

| | | | | | |
|---|---|---|---|---|---|
| Elementaranalyse x 0.5 H₂O | Ber. | C 68.29 | H 6.69 | N 5.31 | S 6.08 |
| | Gef. | C 68.84 | H 6.69 | N 5.32 | S 6.07 |

### 37. (+/-)-cis-8,9-Diethoxy-6-(4-hydroxyphenyl)-1,2,3,4,4a,10b-hexahydrophenanthridin

2,68g (+/-)-cis-6-(4-Acetoxyphenyl)-8,9-diethoxy-1,2,3,4,4a,10b-hexahydrophenanlhridin werden in 15 ml Methanol gelöst, mit 1,1 g Kaliumhydroxid versetzt und 2 h bei RT gerührt. Nach Entfemen des Lösungsmittels im Vakuum wird der Rückstand in Wasser aufgenommen, neutral gestellt und mit Essigsäureethylester extrahiert. Die organische Phase wird mit Natriumsulfat getrocknet und das Lösungsmittel im Vakuum entfernt. Man erhält 1,23 g (51,2 % d.Th.) der Titelverbindung mit Schmp. 232-234°C.
SF: C₂₃ H₂₇ N O₃; MG: 365.48

| | | | | |
|---|---|---|---|---|
| Elementaranalyse (x 0.6 H₂ O) | Ber. | C 73.42 | H 7.55 | N 3.72 |
| | Gef. | C 73.51 | H 7.39 | N 3.79 |

### Ausgangsverbindungen

### A1. (+/-)-cis-N-[2-(3,4-Dimethoxyphenyl)cyclohexyl]-4-p-toluolsulfonamidobenzamid

6,4 g (+/-)-cis-1,2-Dimethoxy-4-(2-aminocyclohexyl)benzol werden in 150 ml Methylenchlorid und 9 ml Triethylamin gelöst. Man tropft bei RT innerhalb von 3 h eine Lösung von 11,2g 4-p-Toluolsulfonamidobenzoesäurechlorid in 200 ml Methylenchlorid zu, extrahiert nach 1 h Rühren mit je 100 ml Wasser, 2N Salzsäure, ges. Natriumhydrogencarbonatlösung und nochmals Wasser. Die organische Phase wird mit Natriumsulfat getrocknet, eingeengt und aus Essigsäureethylester kristallisiert. Man erhält 3,9 g (28,2 % d.Th.) der Titelverbindung mit Schmp. 174-176°C.

Ausgehend von den nachfolgend beschriebenen Ausgangsverbindungen erhält man entsprechend der Arbeitsweise nach Beispiel A1:

### A2. (+/-)-cis-N-[2-(3,4-Diethoxyphenyl)cyclohexyl]-4-p-toluolsulfonamidobenzamid

erstarrendes Öl; Ausbeute 70,6 % d.Th.

### A3. (+/-)-cis-N-[2-(3-Cyclopentyloxy-4-methoxyphenyl)cyclohexyl]-4-p-toluolsulfonamidobenzamid

Öl; Ausbeute 71,6 % d.Th.

### A4. (+/-)-cis-N-[2-(4-Cyclopentyloxy-3-methoxyphenyl)cyclohexyl]-4-p-toluolsulfonamidobenzamid

Schmp.: 90°C, Ausbeute 55,6 % d.Th.

### A5. (+/-)-cis-N-[2-(3,4-Dimethoxyphenyl)cyclohexyl]-4-nitrobenzamid

Schmp.: 122°C, Ausbeute 98,0 % d.Th.

### A6. (+/-)-cis-N-[2-(3,4-Dimethoxyphenyl)cyclohexyl]-2,6-dichlorbenzamid

Schmp.: 181-184.5°C, Ausbeute quantitativ

### A7. (+/-)-cis-N-[2-(3,4-Dimethoxyphenyl)cyclohexyl]-3,4-dimethoxybenzamid

Öl, Ausbeute quantitativ

### A8. (+/-)-cis-N-[2-(3,4-Dimethoxyphenyl)-4,5-dimethylcyclohex-4-enyl]-4-p-toluolsulfonamidobenzamid

Schmp.: 129-141°C, Ausbeute 31,5 % d.Th.

### A9. (+/-)-trans-N-[2-(3,4-Dimethoxyphenyl)cyclohexyl]-4-p-toluolsulfonamidobenzamid

Schmp.: 214-220°C, Ausbeute 34,5 % d.Th.

### A10. (+/-)-trans-N-[2-(3,4-Dimethoxyphenyl)cyclohex-4-enyl]-4-p-toluolsulfonamidobenzamid

Schmp.: 119-126°C, Ausbeute 93,5 % d.Th.

### A11. (+/-)-trans-N-[2-(3,4-Dimethoxyphenyl)-4,5-dimethylcyclohex-4-enyl]-4-p-toluolsulfonamidobenzamid

Schmp.: 139°C (Zersetzung), Ausbeute 64,9 % d.Th.

### A12. (+/-)-trans-N-[2-(3,4-Dimethoxyphenyl)-bicyclo[2.2.2]oct-2-yl-4-ptoluolsulfonamidobenzamid

Öl, Ausbeute 52,5 % d.Th.

### A13. (+/-)-cis-N-[2-(3,4-Diethoxyphenyl)cyclonhexyl]-4-acetoxybenzamid

Schmp.: 81-84°C, Ausbeute 75,8 % d.Th.

### A14. (+/-)-cis-N-[2-(3,4-Diethoxyphenyl)cyclohexyl]-4-benzylbenzamid

Schmp.: 146-150°C, Ausbeute 72,7 % d.Th.

### A15. (+/-)-cis-N-[2-(3,4-Diethoxyphenyl)cyclohexyl]-4-phenylbenzamid

Schmp.: 151-154°C, Ausbeute 48,0 % d.Th.

### A16. (+/-)-cis-N-[2-(3,4-Diethoxyphenyl)cyclohexyl]-4-fluorbenzamid

Schmp.: 149-150°C, Ausbeute 63,0 % d.Th.

### A17. (+/-)-cis-N-[2-(3,4-Diethoxyphenyl)cyclohexyl]-4-trifluormethylbenzamid

Schmp.: 155-156°C, Ausbeute 85,7 % d.Th.

### A18. (+/-)-cis-N-[2-(3,4-Diethoxyphenyl)cyclohexyl]-4-cyanobenzamid

Schmp.: 165-167°C, Ausbeute 75,3 % d.Th.

### A19. (+/-)-cis-N-[2-(3,4-Diethoxyphenyl)cyclohexyl]-4-trifluormethoxybenzamid

Schmp.: 111-113,5°C, Ausbeute 38,9 % d.Th.

### A20. (+/-)-cis-N-[2-(3-Ethoxy-4-methoxyphenyl)cyclohexyl]-4-p-toluolsulfonamidobenzamid

Schmp.: 143-150°C, Ausbeute 60,7 % d.Th.

### A21. (+/-)-cis-N-[2-(3-Ethoxy-4-methoxyphenyl)cyclohexyl]-4-nitrobenzamid

Schmp.: 120-140°C, Ausbeute 89,0 % d.Th.

### A22. (+/-)-cis-N-[2-(3-Ethoxy-4-methoxyphenyl)cyclohexyl]-4-methansulfonylbenzamid

Schmp.: 180-181°C, Ausbeute 75,3 % d.Th.

### A23. (+/-)-cis-N-[2-(3-Ethoxy-4-methoxyphenyl)cyclohexyl]-4-(4-methoxycarbonylphenyl)sulfonamidobenzamid

Schmp.: 176-182°C, Ausbeute 42,4 % d.Th.

### A24. (+/-)-cis-N-[2-(3,4-Diethoxyphenyl)cyclohexyl]-4-isonicotinsäureamid

2,5 g (+/-)-cis-1,2-Diethoxy-4-(2-aminocyclohexyl)benzol werden in 30 ml Methylenchlorid und 5 ml Triethylamin gelöst. Man tropft bei RT innerhalb von 3 h eine Suspension von 2,0 g Isonicotinsäurechlorid in 30 ml Meihylenchlorid zu, extrahiert nach 1 h Rühren mit je 50 ml Wasser, 2N Salzsäure, ges. Natriumhydrogencarbonatlösung und nochmals Wasser. Die organische Phase wird mit Natriumsulfat getrocknet, eingeengt und der Rückstand über Kieselgel mit einer Mischung aus Toluol/Dioxan/-Petroläther/Triethylamin im Verhältnis 6:2:1:0.5 chromatographiert. Nach Einengen der entsprechenden Eluate erhält man 3,44 g (98,3 % d.Th.) der Titelverbindung als erstarrendes Öl.

Ausgehend von den nachfolgend beschriebenen Ausgangsverbindungen erhält man entsprechend der Arbeitsweise nach Beispiel A24:

### A25. (+/-)-cis-N-[2-(3,4-Diethoxyphenyl)cyclohexyl]-4-nicotinsäureamid

erstarrendes Öl; Ausbeute quantitativ

### A26. (+/-)-cis-N-[2-(3,4-Diethoxyphenyl)cyclohexyl]-6-chlornicotinsäureamid

Öl; Ausbeute 59,0 % d.Th.

### B1. (+/-)-cis-1,2-Dimethoxy-4-(2-aminocyclohexyl)benzol

8,5 g (+/-)-cis-1,2-Dimethoxy-4-(2-nitrocyclohexyl)benzol werden in 400 ml Methanol gelöst und bei RT innerhalb von 8 h portionsweise mit 7 ml Hydrazinhydrat und 2,5 g Raney-Nickel versetzt. Nach Rühren über Nacht bei RT wird das Reaktionsgemisch filtriert, das Filtrat eingeengt und der Rückstand über Kieselgel mit einer Mischung aus Toluol/Essigsäureethylester/Triethylamin = (4:2:0.5) chromatographiert.

Öl; Ausbeute 74,4 % d.Th.

Ausgehend von den nachfolgend beschriebenen Ausgangsverbindungen erhält man entsprechend der Arbeitsweise nach Beispiel B1:

### B2. (+/-)-cis-1,2-Diethoxy-4-[2-aminocyclohexyl)benzol

Öl; Ausbeute 42,8 % d.Th.

### B3. (+/-)-cis-2-Cyclopentyloxy-1-methoxy-4-(2-aminocyclohexyl)benzol

Öl; Ausbeute 68,2 % d.Th.

### B4. (+/-)-cis-1-Cyclopentyloxy-2-methoxy-4-(2-aminocyclohexyl)benzol

Öl, Ausbeute 69,0 % d.Th.

### B5. (+/-)-cis-1,2-Dimethoxy-4-(2-amino-4,5-dimethylcyclohex-4-enyl)benzol

Öl, Ausbeute 87,3 % d.Th.

### B6. (+/-)-trans-1,2-Dimethoxy-4-(2-aminocyclohexyl)benzol

Öl, Ausbeute 65,9 % d.Th.

### B7. (+/-)-trans-1,2-Dimethoxy-4-(2-aminocyclohex-4-enyl)benzol

Öl, Ausbeute 28,9 % d.Th.

### B8. (+/-)-trans-1,2-Dimethoxy-4-(2-amino-4,5-dimethylcyclohex-4-enyl)benzol

erstarrtes Öl, Ausbeute 94 % d.Th.

### B9. (+/-)-trans-3-(3,4-Dimethoxyphenyl)-bicyclo[2.2.2]oct-2-vlamin

Öl, Ausbeute 70,7 % d.Th.

### B10. (+/-)-cis-2-Ethoxy-1-methoxy-4-(2-aminocyclohexyl)benzol

40,0 g (+/-)-cis-2-Ethoxy-1-methoxy-4-(2-nitrocyclohex-4-enyl)benzol werden in 1000 ml Ethanol und 500 ml Tetrahydrofuran gelöst, mit 10 g Raney Nickel versetzt und bei 100 bar Wasserstoffdruck 4 Tage im Autoklaven bei RT hydriert. Nach Filtration und Entfemen des Lösungsmittels im Vakuum erhält man 35,9 g (99,8 % d.Th.) der Titelverbindung als erstarrendes Öl.

### C1. (+/-)-cis-1,2-Dimethoxy-4-(2-nitrocyclohexyl)benzol

8,4 g (+/-)-cis-1,2-Dimethoxy-4-(2-nitrocyclohex-4-enyl)benzol werden in 450 ml Methanol gelöst, mit 2 ml konz. Salzsäure versetzt und nach Zugabe von 500 mg Pd/C 10 %ig hydriert. Das Reaktionsgemisch wird filtriert und das Filtrat eingeengt. Schmp.: 84-86,5°C; Ausbeute quantitativ.

Ausgehend von den nachfolgend beschriebenen Ausgangsverbindungen erhält man entsprechend der Arbeitsweise nach Beispiel C1:

### C2. (+/-)-cis-1,2-Diethoxy-4-(2-nitrocyclohexyl)benzol

Öl; Ausbeute 96,5 % d.Th.

### C3. (+/-)-cis-2-Cyclopentyloxy-1-methoxy-4-(2-nitrocyclohexyl)benzol

Schmp.: 107,5°C, Ausbeute 53,5 % d.Th.

### C4. (+/-)-cis-1-Cyclopentyloxy-2-methoxy-4-(2-nitrocyclohexyl)benzol

Schmp.: 92-94,5°C, Ausbeute 74,8 % d.Th.

### C5. (+/-)-trans-1,2-Dimethoxy-4-(2-nitrocyclohexyl)benzol

Öl, Ausbeute 47,0 % d.Th.

### C6. (+/-)-trans-3-(3,4-Dimethoxyphenyl)-2-nitrobicyclo[2.2.2]octan

Öl, Ausbeute 76,0 % d.Th.

### D1. (+/-)-cis-1,2-Dimethoxy-4-(2-nitrocyclohex-4-enyl)benzol

10,09 (+/-)-trans-1,2-Dimethoxy-4-(2-nitrocyclohex-4-enyl)benzol und 20,0 g Kaliumhydroxid werden in 150 ml Ethanol und 35 ml Dimethylformamid gelöst. Anschließend wird eine Lösung von 17,5 ml konz. Schwefelsäure in 60 ml Ethanol so zugetropft, daß die Innentemperatur 4°C nicht übersteigt. Nach 1 h Rühren wird auf 1 I Eiswasser gegeben, der Niederschlag abgesaugt, mit Wasser gewaschen. getrocknet und das Rohprodukt aus Ethanol umkristallisiert. Schmp.: 82,5-84°C; Ausbeute 86 % d.Th.

Ausgehend von den nachfolgend beschriebenen Ausgangsverbindungen erhält man entsprechend der Arbeitsweise nach Beispiel D1:

### D2. (+/-)-cis-1,2-Diethoxy-4-(2-nitrocyclohex-4-enyl)benzol

Öl; Ausbeute 96,5 % d.Th.

### D3. (+/-)-cis-2-Cyclopentyloxy-1-methoxy-4-(2-nitrocyclohex-4-enyl)benzol

Schmp.: 78-81°C, Ausbeute 89,2 % d.Th.

### D4. (+/-)-cis-1-Cyclopentyloxy-2-methoxy-4-(2-nitrocyclohex-4-enyl)benzol

Schmp.: 81,5-85°C, Ausbeute quantitativ

### D5. (+/-)-cis-2-Ethoxy-1-methoxy-4-(2-nitrocyclohex-4-enyl)benzol

Schmp.: 66-67°C, Ausbeute 97,2 % d.Th.

### D6. (+/-)-cis-1,2-Dimethoxy-4-(4,5-dimethyl-2-nitrocyclohex-4-enyl)benzol

Schmp.: 97,5°C, Ausbeute 91,8 % d.Th.

### E1. (+/-)-trans-1,2-Dimethoxy-4-(2-nitrocyclohex-4-enyl)benzol

50,0 g 3,4-Dimethoxy-ω-nitrostyrol und 1,0g (9,1 mmol) Hydrochinon werden in 200 ml abs. Toluol suspendiert und bei -70°C mit 55,0 g (1,02 mol) flüssigem 1,3-Butadien versetzt. Die Mischung wird im Autoklaven 6 Tage bei 160°C gerührt und dann abgekühlt. Ein Teil des Lösungsmittels wird im Vakuum abdestilliert, der entstehende Niederschlag wird abgesaugt und in Ethanol umkristallisiert. Schmp.: 113.5-115,5°C; Ausbeute 76,3 % d.Th.

Ausgehend von den nachfolgend beschriebenen Ausgangsverbindungen erhält man entsprechend der Arbeitsweise nach Beispiel E1:

### E2. (+/-)-trans-1,2-Diethoxy-4-(2-nitrocyclohex-4-enyl)benzol

Schmp.: 80-81,5°C; Ausbeute 59,8 % d.Th.

### E3. (+/-)-trans-2-Cyclopentyloxy-1-methoxy-4-(2-nitrocyclohex-4-enyl)benzol

Schmp.: 135-136°C; Ausbeute 77,7 % d.Th.

### E4. (+/-)-trans-1-Cyclopentyloxy-2-methoxy-4-(2-nitrocyclohex-4-enyl)benzol

Schmp.: 109°C; Ausbeute 71,1 % d.Th.

### E5. (+/-)-trans-2-Ethoxy-1-methoxy-4-(2-nitrocyclohex-4-enyl)benzol

Schmp.: 129-130°C, Ausbeute 75,7 % d.Th.

### E6. (+/-)-trans-1,2-Dimethoxy-4-(4,5-dimethyl-2-nitrocyclohex-4-enyl)benzol

Schmp.: 131,5°C; Ausbeute 79,3 % d.Th.

### E7. (+/-)-trans-5-(3,4-Dimethoxyphenyl)-6-nitrobicyclo[2,2,2]oct-2-ene

Öl, Ausbeute quantitativ

### F1. 3,4-Dimethoxy-ω-nitrostyrol

207,0 g 3,4-Dimethoxybenzaldehyd, 100,0 g Ammoniumacetat und 125 ml Nitromethan werden in 1,0 I Eisessig 3-4 h zum Sieden erhitzt. Nach Abkühlen im Eisbad wird der Niederschlag abgesaugt, mit Eisessig und Petroläther nachgespült und getrocknet. Schmp.: 140-141°C. Ausbeute: 179,0 g (68,5 % d.Th.)

Ausgehend von entsprechenden Ausgangsverbindungen der Formel VII erhält man analog der Arbeitsweise nach Beispiel F1:

### F2. 3,4-Diethoxy-ω-nitrostyrol

Schmp.: 136-136,5°C; Ausbeute: 76,2 % d.Th.

### F3. 3-Cyclopentyloxy-4-methoxy-ω-nitrostyrol

Schmp.: 137-138°C; Ausbeute: 86,6 % d.Th.

### F4. 4-Cyclopentyloxy-3-methoxy-ω-nitrostyrol

Schmp.: 90-91°C; Ausbeute: 44,0 % d.Th.

### F5. 3-Ethoxy-4-methoxy-ω-nitrostyrol

Schmp.: 132-133°C, Ausbeute 70,3 % d.Th.

### Gewerbliche Anwendbarkeit

Die erfindungsgemäßen Verbindungen besitzen wertvolle pharmakologische Eigenschaften, die sie gewerblich verwertbar machen. Als selektive Zyklisch-Nukleotid Phosphodiesterase (PDE) Inhibitoren (und zwar des Typs IV) eignen sie sich einerseits als Bronchialtherapeutika (zur Behandlung von Atemwegsobstruktionen aufgrund ihrer dilatierenden aber auch aufgrund ihrer atemfrequenz- bzw. atemantriebssteigemden Wirkung) und zur Behebung von erektiler Dysfunktion aufgrund der gefäßdilatierenden Wirkung, andererseits jedoch vor allem zur Behandlung von Erkrankungen, insbesondere entzündlicher Natur, z.B. der Atemwege (Asthma-Prophylaxe), der Haut, des Darms, der Augen, des zentralen Nervensystems und der Gelenke, die vermittelt werden durch Mediatoren, wie Histamin, PAF (Plättchen- aktivierender Faktor), Arachidonsäure-Abkömmlinge wie Leukotriene und Prostaglandine, Zytokine, Interleukine, Chemokine, alpha-, beta- und gamma-lnterferon, Tumomekrosisfaktor (TNF) oder Sauerstoff-Radikale und Proteasen. Hierbei zeichnen sich die erfindungsgemäßen Verbindungen durch eine geringe Toxizität, eine gute enterale Resorption (hohe Bioverfügbarkeit), eine große therapeutische Breite und das Fehlen wesentlicher Nebenwirkungen aus.

Aufgrund ihrer PDE-hemmenden Eigenschaften können die erfindungsgemäßen Verbindungen in der Human- und Veterinärmedizin als Therapeutika eingesetzt werden, wobei sie beispielsweise zur Behandlung und Prophylaxe folgender Krankheiten verwendet werden können: Akute und chronische (insbesondere entzündliche und allergeninduzierte) Atemwegserkrankungen verschiedener Genese (Bronchitis, allergische Bronchitis, Asthma bronchiale); Dermatosen (vor allem proliferativer, entzündlicher und allergischer Art) wie beispielsweise Psoriasis (vulgaris), toxisches und allergisches Kontaktekzem, atopisches Ekzem, seborrhoisches Ekzem, Lichen simplex, Sonnenbrand, Pruritus im Genitoanalbereich, Alopecia areata, hypertrophe Narben, diskoider Lupus erythematodes, follikuläre und flächenhafte Pyodermien, endogene und exogene Akne, Akne rosacea sowie andere proliferative, entzündliche und allergische Hauterkrankungen; Erkrankungen, die auf einer überhöhten Freisetzung von TNF und Leukotrienen beruhen, so z.B. Erkrankungen aus dem Formenkreis der Arthritis (Rheumatoide Arthritis, Rheumatoide Spondylitis, Osteoarthritis und andere arthritische Zustände), Erkrankungen des Immunsystems (AIDS, Multiple Sklerose), Erscheinungsformen des Schocks [septischer Schock, Endotoxinschock, gram-negative Sepsis, Toxisches Schock-Syndrom und das ARDS (adult respiratory distress syndrom)] sowie generalisierte Entzündungen im Magen-Darm Bereich (Morbus Crohn und Colitis ulcerosa); Erkrankungen, die auf allergischen und/oder chronischen, immunologischen Fehlreaktionen im Bereich der oberen Atemwege (Rachenraum, Nase) und der angrenzenden Regionen (Nasennebenhöhlen, Augen) beruhen, wie beispielsweise allergische Rhinitis/Sinusitis, chronische Rhinitis/Sinusitis, allergische Conjunctivitis sowie Nasenpolypen; aber auch Erkrankungen des Herzens, die durch PDE-Hemmstoffe behandelt werden können, wie beispielsweise Herzinsuffizienz, oder Erkrankungen, die aufgrund der gewebsrelaxierenden Wirkung der PDE-Hemmstoffe behandelt werden können, wie beispielsweise erektile Dysfunktion oder Koliken der Nieren und der Harnleiter im Zusammenhang mit Nierensteinen.

Ein weiterer Gegenstand der Erfindung ist ein Verfahren zur Behandlung von Säugetieren einschließlich Menschen, die an einer der oben genannten Krankheiten erkrankt sind. Das Verfahren ist dadurch gekennzeichnet, daß man dem erkrankten Säugetier eine therapeutisch wirksame und pharmakologisch verträgliche Menge einer oder mehrerer der erfindungsgemäßen Verbindungen verabreicht.

Weiterer Gegenstand der Erfindung sind die erfindungsgemäßen Verbindungen zur Anwendung bei der Behandlung und/oder Prophylaxe der genannten Krankheiten.

Ebenso betrifft die Erfindung die Verwendung der erfindungsgemäßen Verbindungen zur Herstellung von Arzneimitteln, die zur Behandlung und/oder Prophylaxe der genannten Krankheiten eingesetzt werden.

Weiterhin sind Arzneimittel zur Behandlung und/oder Prophylaxe der genannten Krankheiten, die eine oder mehrere der erfindungsgemäßen Verbindungen enthalten, Gegenstand der Erfindung.

Die Arzneimittel werden nach an sich bekannten, dem Fachmann geläufigen Verfahren hergestellt. Als Arzneimittel werden die erfindungsgemäßen Verbindungen (= Wirkstoffe) entweder als solche, oder vorzugsweise in Kombination mit geeigneten pharmazeutischen Hilfsstoffen z.B. in Form von Tabletten, Dragees, Kapseln, Suppositorien, Pflastern, Emulsionen, Suspensionen, Gelen oder Lösungen eingesetzt, wobei der Wirkstoffgehalt vorteilhafterweise zwischen 0,1 und 95 % beträgt.

Welche Hilfsstoffe für die gewünschten Arzneiformulierungen geeignet sind, ist dem Fachmann aufgrund seines Fachwissens geläufig. Neben Lösemitteln, Gelbildnem, Salbengrundlagen und anderen Wirkstoffträgem können beispielsweise Antioxidantien, Dispergiermittel, Emulgatoren, Konservierungsmittel, Lösungsvermittler oder Permeationspromotoren verwendet werden.

Für die Behandlung von Erkrankungen des Respirationstraktes werden die erfindungsgemäßen Verbindungen bevorzugt auch inhalativ appliziert. Hierzu werden diese entweder direkt als Pulver (vorzugsweise in mikronisierter Form) oder durch Vernebeln von Lösungen oder Suspensionen, die sie enthalten, verabreicht. Bezüglich der Zubereitungen und Darreichungsformen wird beispielsweise auf die Ausführungen im Europäischen Patent 163 965 verwiesen.

Für die Behandlung von Dermatosen erfolgt die Anwendung der erfindungsgemäßen Verbindungen insbesondere in Form solcher Arzneimittel, die für eine topische Applikation geeignet sind. Für die Herstellung der Arzneimittel werden die erfindungsgemäßen Verbindungen (= Wirkstoffe) vorzugsweise mit geeigneten pharmazeutischen Hilfsstoffen vermischt und zu geeigneten Arzneiformulierungen weiterverarbeitet. Als geeignete Arzneiformulierungen seien beispielsweise Puder, Emulsionen, Suspensionen, Sprays, Öle, Salben, Fettsalben, Cremes, Pasten, Gele oder Lösungen genannt.

Die erfindungsgemäßen Arzneimittel werden nach an sich bekannten Verfahren hergestellt. Die Dosierung der Wirkstoffe erfolgt in der für PDE-Hemmstoffe üblichen Größenordnung. So enthalten topische Applikationsformen (wie z.B. Salben) für die Behandlung von Dermatosen die Wirkstoffe in einer Konzentration von beispielsweise 0,1-99 %. Die Dosis für die inhalative Applikation beträgt üblicherweise zwischen 0,01 und 1 mg pro Sprühstoß. Die übliche Dosis bei systemischer Therapie p.o. oder i.v. liegt zwischen 0,1 und 200 mg pro Applikation.

### Biologische Untersuchungen

Bei der Untersuchung der PDE IV-Hemmung auf zellulärer Ebene kommt der Aktivierung von Entzündungszellen besondere Bedeutung zu. Als Beispiel sei die FMLP (N-formyl-methionyl-leucylphenylalanin)-induzierte Superoxid-Produktion von neutrophilen Granulozyten genannt, die als Luminol-verstärkte Chemolumineszenz gemessen werden kann. [Mc Phail LC, Strum SL, Leone PA und Sozzani S, The neutrophil respiratory burst mechanism. In "Immunology Series" **1992**, 57, 47-76; ed. Coffey RG (Marcel Decker, Inc., New York-Basel-Hong Kong)].

Substanzen, welche die Chemolumineszenz sowie die Zytokinsekretion und die Sekretion entzündungssteigemder Mediatoren an Entzündungszellen, insbesondere die neutrophilen und eosinophilen Granulozyten, die T-Lymphozyten, die Monozyten und die Macrophagen hemmen, sind solche, welche die PDE IV hemmen. Dieses Isoenzym der Phosphodiesterase-Familien ist besonders in Granulozyten vertreten. Dessen Hemmung führt zur Erhöhung der intrazellulären zyklischen AMP-Konzentration und damit zur Hemmung der zellulären Aktivierung. Die PDE IV-Hemmung durch die erfindungsgemäßen Substanzen ist damit ein zentraler Indikator für die Unterdrückung von entzündlichen Prozessen. (Giembycz MA, Could isoenzyme-selective phosphodiesterase inhibitors render bronchodilatory therapy redundant in the treatment of bronchial asthma?. Biochem Pharmacol **1992**, 43, 2041-2051; Torphy TJ et al., Phosphodiesterase inhibitors: new opportunities for treatment of asthma. Thorax **1991**, 46, 512-523; **Schudt C** et al., Zardaverine: a cyclic AMP PDE III/IV inhibitor. In "New Drugs for Asthma Therapy", 379-402, Birkhäuser Verlag Basel 1991; **Schudt C** et al., Influence of selective phosphodiesterase inhibitors on human neutrophil functions and levels of cAMP and Caᵢ. Naunyn-Schmiedebergs Arch Pharmacol **1991**, 344, 682-690; **Nielson CP** et al., Effects of selective phosphodiesterase inhibitors on polymorphonuclear leukocyte respiratory burst. J Allergy Clin Immunol **1990**, 86, 801-808; **Schade** et al., The specific type III and IV phosphodiesterase inhibitor zardaverine suppress formation of tumor necrosis factor by macrophages. European Journal of Pharmacology **1993**, 230, 9-14).

### 1. Hemmung der PDE IV-Aktivität

### Methodik

Der Aktivitätstest wurde nach der Methode von Bauer und Schwabe durchgeführt, die auf Mikrotiterplatten adaptiert wurde (Naunyn-Schmiedeberg's Arch. Pharmacol. **1980**, 311, 193-198). Hierbei erfolgt im ersten Schritt die PDE-Reaktion. In einem zweiten Schritt wird das entstandene 5'-Nukleotid durch eine 5'-Nukleotidase des Schlangengiftes von Crotalus Atrox zum ungeladenen Nukleosid gespalten. Im dritten Schritt wird das Nukleosid auf lonenaustauschsäulen vom verbliebenen geladenen Substrat getrennt. Die Säulen werden mit 2 ml 30 mM Ammoniumformiat (pH 6,0) direkt in Minivials eluiert, in die noch 2 ml Szintillatorflüssigkeit zur Zählung gegeben wird.

Die für die erfindungsgemäßen Verbindungen ermittelten Hemmwerte ergeben sich aus der folgenden Tabelle A, in der die Nummern der Verbindungen den Nummern der Beispiele entsprechen.

**Tabelle A**

| **Hemmung der PDE IV-Aktivität** | |
|---|---|
| **Verbindung** | **-log IC**_{**50**} |
| 1 | 7.73 |
| 2 | 8.39 |
| 3 | 7.76 |
| 4 | 6.12 |
| 5 | 7.22 |
| 6 | 6.77 |
| 7 | 6.44 |
| 8 | 6.91 |
| 9 | 6.17 |
| 10 | 6.10 |
| 12 | 5.77 |
| 13 | 8.43 |
| 14 | 8.08 |
| 15 | 7.98 |
| 16 | 7.93 |
| 17 | 8.00 |
| 18 | 8.24 |
| 19 | 7.97 |
| 20 | 9.05 |
| 21 | 8.46 |
| 22 | 8.66 |
| 23 | 8.70 |
| 24 | 8.08 |
| 25 | 7.96 |
| 26 | 8.41 |
| 27 | 6.66 |
| 29 | 7.69 |
| 30 | 7.36 |
| 31 | 7.30 |
| 32 | 8.38 |
| 33 | 7.27 |
| 34 | 7.42 |
| 35 | 7.81 |
| 36 | 9.09 |
| 37 | 8.04 |

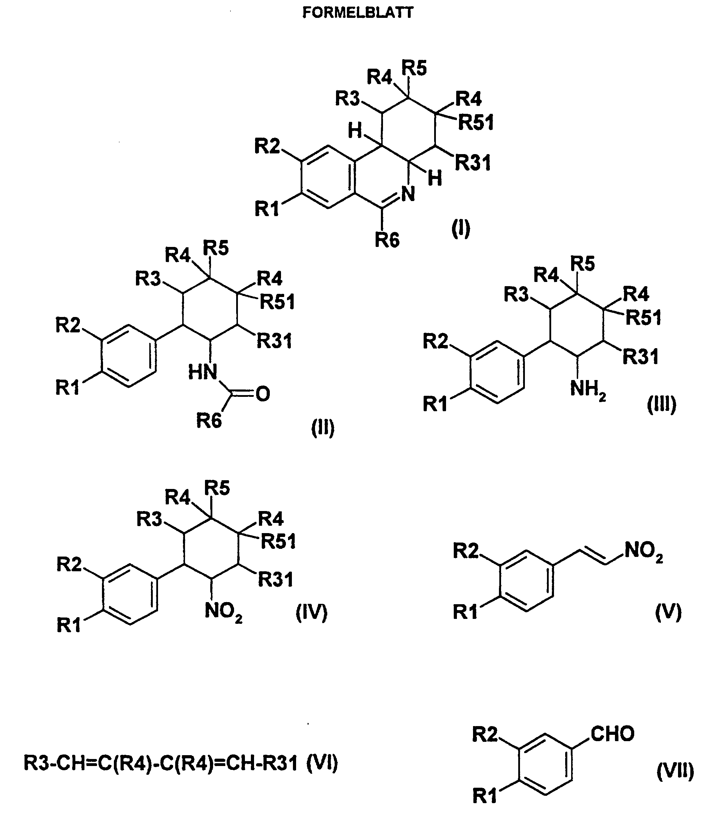

## Patentansprüche

1. Verbindungen der Formel I, 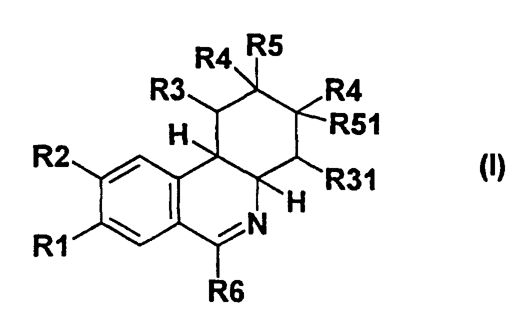 worin
R1 Hydroxy, 1-4C-Alkoxy, 3-7C-Cycloalkoxy, 3-7C-Cycloalkylmethoxy oder ganz oder teilweise durch Fluor substituiertes 1-4C-Alkoxy bedeutet,
R2 Hydroxy, 1-4C-Alkoxy, 3-7C-Cycloalkoxy, 3-7C-Cycloalkytmethoxy oder ganz oder teilweise durch Fluor substituiertes 1-4C-Alkoxy bedeutet,
oder worin
R1 und R2 gemeinsam eine 1-2C-Alkylendioxygruppe bedeuten,
R3 Wasserstoff oder 1-4C-Alkyl bedeutet,
R31 Wasserstoff oder 1-4C-Alkyl bedeutet,
oder worin
R3 und R31 gemeinsam eine 1-4C-Alkylengruppe bedeuten,
R4 Wasserstoff oder 1-4C-Alkyl bedeutet,
R5 Wasserstoff bedeutet,
R51 Wasserstoff bedeutet,
oder worin
R5 und R51 gemeinsam eine zusätzliche Bindung darstellen,
R6 einen durch R61 substituierten Pyridylrest darstellt oder einen durch R7 und R8 substituierten Phenylrest darstellt, wobei
R61 Wasserstoff, Hydroxy, Halogen, 1-4C-Alkoxy, 1-4C-Alkyl, Carboxyl, Trifluormethyl, 1-4C-Alkoxycarbonyl oder ganz oder teilweise durch Fluor substituiertes 1-4C-Alkoxy bedeutet,
R7 Hydroxy, Halogen, Cyano, 1-4C-Alkyl, 1-4C-Alkoxy, 1-4C-Alkylcarbonyloxy, Trifluormethyl, Phenyl, Phenyl-1-4C-alkyl, Nitro, Amino, ganz oder teilweise durch Fluor substituiertes 1-4C-Alkoxy, SO₂-R70 oder N(R71)R72 bedeutet, wobei
R70 1-4C-Alkyl,
R71 Wasserstoff, 1-4C-Alkyl, SO₂-R9 oder SO₂-R10 und
R72 1-4C-Alkyl, 1-4C-Alkylcarbonyl oder SO₂-R10 bedeutet,
R8 Wasserstoff, Hydroxy, Halogen, 1-4C-Alkoxy oder 1-4C-Alkyl bedeutet
und wobei
R9 und R10 unabhängig voneinander 1-4C-Alkyl, Phenyl, Phenyl-1-4C-alkyl oder durch einen oder mehrere gleiche oder verschiedene Substituenten substituiertes Phenyl bedeuten, wobei die Substituenten ausgewählt sind aus der Gruppe Nitro, 1-4C-Alkyl, Halogen, 1-4C-Alkylcarbonylamino, 1-4C-Alkoxy, ganz oder teilweise durch Fluor substituiertes 1-4C-Alkoxy, Cyano, Phenyl, Naphthyl, Trifluormethyl oder 1-4C-Alkoxycarbonyl,
sowie die Salze dieser Verbindungen.

2. Verbindungen der Formel I nach Anspruch 1, worin
R1 Hydroxy, 1-4C-Alkoxy, 3-7C-Cycloalkoxy, 3-7C-Cycloalkylmethoxy oder ganz oder teilweise durch Fluor substituiertes 1-4C-Alkoxy bedeutet,
R2 Hydroxy, 1-4C-Alkoxy, 3-7C-Cycloalkoxy, 3-7C-Cycloalkylmethoxy oder ganz oder teilweise durch Fluor substituiertes 1-4C-Alkoxy bedeutet,
oder worin
R1 und R2 gemeinsam eine 1-2C-Alkylendioxygruppe bedeuten,
R3 Wasserstoff oder 1-4C-Alkyl bedeutet,
R31 Wasserstoff oder 1-4C-Alkyl bedeutet,
oder worin
R3 und R31 gemeinsam eine 1-4C-Alkylengruppe bedeuten,
R4 Wasserstoff oder 1-4C-Alkyl bedeutet,
R5 Wasserstoff bedeutet,
R51 Wasserstoff bedeutet,
oder worin
R5 und R51 gemeinsam eine zusätzliche Bindung darstellen,
R6 einen durch R61 substituierten Pyridylrest darstellt, wobei
R61 Wasserstoff, Hydroxy, Halogen, 1-4C-Alkoxy, 1-4C-Alkyl, Carboxyl, Trifluormethyl, 1-4C-Alkoxycarbonyl oder ganz oder teilweise durch Fluor substituiertes 1-4C-Alkoxy bedeutet,
sowie die Salze dieser Verbindungen.

3. Verbindungen der Formel I nach Anspruch 1, worin
R1 Hydroxy, 1-4C-Alkoxy, 3-7C-Cycloalkoxy, 3-7C-Cycloalkylmethoxy oder ganz oder teilweise durch Fluor substituiertes 1-4C-Alkoxy bedeutet,
R2 Hydroxy, 1-4C-Alkoxy, 3-7C-Cycloalkoxy, 3-7C-Cycloalkylmethoxy oder ganz oder teilweise durch Fluor substituiertes 1-4C-Alkoxy bedeutet,
oder worin
R1 und R2 gemeinsam eine 1-2C-Alkylendioxygruppe bedeuten,
R3 Wasserstoff oder 1-4C-Alkyl bedeutet,
R31 Wasserstoff oder 1-4C-Alkyl bedeutet,
oder worin
R3 und R31 gemeinsam eine 1-4C-Alkylengruppe bedeuten,
R4 Wasserstoff oder 1-4C-Alkyl bedeutet,
R5 Wasserstoff bedeutet,
R51 Wasserstoff bedeutet,
oder worin
R5 und R51 gemeinsam eine zusätzliche Bindung darstellen,
R6 einen durch R7 und R8 substituierten Phenylrest darstellt, wobei
R7 Hydroxy, Halogen, Cyano, 1-4C-Alkyl, 1-4C-Alkoxy, 1-4C-Alkylcarbonyloxy, Trifluormethyl, Phenyl, Phenyl-1-4C-alkyl, Nitro, Amino oder N(R71)R72 bedeutet, wobei
R71 Wasserstoff, 1-4C-Alkyl, SO₂-R9 oder SO₂-R10 und
R72 1-4C-Alkyl, 1-4C-Alkylcarbonyl oder SO₂-R10 bedeutet,
R8 Wasserstoff, Hydroxy, Halogen, 1-4C-Alkoxy oder 1-4C-Alkyl bedeutet
und wobei
R9 und R10 unabhängig voneinander 1-4C-Alkyl, Phenyl, Phenyl-1-4C-alkyl oder durch einen oder mehrere gleiche oder verschiedene Substituenten substituiertes Phenyl bedeuten, wobei die Substituenten ausgewählt sind aus der Gruppe Nitro, 1-4C-Alkyl, Halogen, 1-4C-Alkylcarbonylamino, 1-4C-Alkoxy, ganz oder teilweise durch Fluor substituiertes 1-4C-Alkoxy, Cyano, Phenyl, Naphthyl oder Trifluormethyl,
sowie die Salze dieser Verbindungen.

4. Verbindungen der Formel I nach Anspruch 1, worin
R1 Hydroxy, 1-4C-Alkoxy, 3-7C-Cycloalkoxy, 3-7C-Cycloalkylmethoxy oder ganz oder teilweise durch Fluor substituiertes 1-4C-Alkoxy bedeutet,
R2 Hydroxy, 1-4C-Alkoxy, 3-7C-Cycloalkoxy, 3-7C-Cycloalkylmethoxy oder ganz oder teilweise durch Fluor substituiertes 1-4C-Alkoxy bedeutet,
oder worin
R1 und R2 gemeinsam eine 1-2C-Alkylendioxygruppe bedeuten,
R3 Wasserstoff oder 1-4C-Alkyl bedeutet,
R31 Wasserstoff oder 1-4C-Alkyl bedeutet,
oder worin
R3 und R31 gemeinsam eine 1-4C-Alkylengruppe bedeuten,
R4 Wasserstoff oder 1-4C-Alkyl bedeutet,
R5 Wasserstoff bedeutet,
R51 Wasserstoff bedeutet,
oder worin
R5 und R51 gemeinsam eine zusätzliche Bindung darstellen,
R6 einen durch R7 und R8 substituierten Phenylrest darstellt, wobei
R7 Hydroxy, Halogen, Cyano, 1-4C-Alkyl, 1-4C-Alkoxy, 1-4C-Alkylcarbonyloxy, Trifluormethyl, Phenyl, Phenyl-1-4C-alkyl, Nitro, Amino, ganz oder teilweise durch Fluor substituiertes 1-4C-Alkoxy, SO₂-R70 oder N(R71)R72 bedeutet, wobei
R70 1-4C-Alkyl,
R71 Wasserstoff, 1-4C-Alkyl, SO₂-R9 oder SO₂-R10 und
R72 1-4C-Alkyl, 1-4C-Alkylcarbonyl oder SO₂-R10 bedeutet,
R8 Wasserstoff, Hydroxy, Halogen, 1-4C-Alkoxy oder 1-4C-Alkyl bedeutet
und wobei
R9 und R10 unabhängig voneinander 1-4C-Alkyl, Phenyl, Phenyl-1-4C-alkyl oder durch einen oder mehrere gleiche oder verschiedene Substituenten substituiertes Phenyl bedeuten, wobei die Substituenten ausgewählt sind aus der Gruppe Nitro, 1-4C-Alkyl, Halogen, 1-4C-Alkylcarbonylamino, 1-4C-Alkoxy, ganz oder teilweise durch Fluor substituiertes 1-4C-Alkoxy, Cyano, Phenyl, Naphthyl, Trifluormethyl oder 1-4C-Alkoxycarbonyl,
sowie die Salze dieser Verbindungen.

5. Verbindungen der Formel I nach Anspruch 1, worin
R1 1-4C-Alkoxy oder 3-7C-Cycloalkoxy bedeutet,
R2 1-4C-Alkoxy oder 3-7C-Cycloalkoxy bedeutet,
R3 Wasserstoff bedeutet,
R31 Wasserstoff bedeutet,
oder worin
R3 und R31 gemeinsam eine 1-2C-Alkylengruppe bedeuten,
R4 Wasserstoff oder 1-4C-Alkyl bedeutet,
R5 Wasserstoff bedeutet,
R51 Wasserstoff bedeutet,
oder worin
R5 und R51 gemeinsam eine zusätzliche Bindung darstellen,
R6 einen durch R7 und R8 substituierten Phenylrest darstellt, wobei
R7 Hydroxy, Halogen, Cyano, 1-4C-Alkoxy, 1-4C-Alkylcarbonyloxy, Trifluormethyl, Phenyl, Phenyl-1-4C-alkyl, Nitro, Amino, ganz oder teilweise durch Fluor substituiertes 1-4C-Alkoxy, SO₂-R70 oder N(R71)R72 bedeutet, wobei
R70 1-4C-Alkyl,
R71 Wasserstoff, 1-4C-Alkyl oder SO₂-R10 und
R72 1-4C-Alkylcarbonyl oder SO₂-R10 bedeutet,
R8 Wasserstoff, Halogen oder 1-4C-Alkoxy bedeutet
und wobei
R10 durch einen Substituenten substituiertes Phenyl bedeutet, wobei der Substituent ausgewählt ist aus der Gruppe Nitro, 1-4C-Alkyl oder 1-4C-Alkoxycarbonyl,
sowie die Salze dieser Verbindungen.

6. Arzneimittel enthaltend mindestens eine Verbindung der Formel I nach Anspruch 1 zusammen mit pharmazeutischen Hilfs- und/oder Trägerstoffen.

7. Verwendung von Verbindungen der Formel I nach Anspruch 1 zur Herstellung von Arzneimitteln für die Behandlung von Atemwegserkrankungen.

## Claims

1. Compounds of formula I 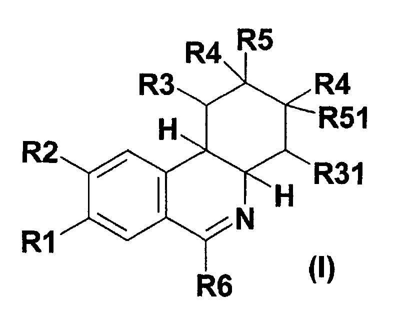 in which
R1 is hydroxyl, 1-4C-alkoxy, 3-7C-cycloalkoxy, 3-7C-cycloalkylmethoxy or 1-4C-alkoxy which is completely or partially substituted by fluorine,
R2 is hydroxyl, 1-4C-alkoxy, 3-7C-cycloalkoxy, 3-7C-cycloalkylmethoxy or 1-4C-alkoxy which is completely or partially substituted by fluorine,
or in which
R1 and R2 together are a 1-2C-alkylenedioxy group,
R3 is hydrogen or 1-4C-alkyl,
R31 is hydrogen or 1-4C-alkyl,
or in which
R3 and R31 together are a 1-4C-alkylene group,
R4 is hydrogen or 1-4C-alkyl,
R5 is hydrogen,
R51 is hydrogen,
or in which
R5 and R51 together are an additional bond,
R6 is a pyridyl radical which is substituted by R61 or a phenyl radical which is substituted by R7 and R8, where
R61 is hydrogen, hydroxyl, halogen, 1-4C-alkoxy, 1-4C-alkyl, carboxyl, trifluoromethyl, 1-4C-alkoxycarbonyl or 1-4C-alkoxy which is completely or partially substituted by fluorine,
R7 is hydroxyl, halogen, cyano, 1-4C-alkyl, 1-4C-alkoxy, 1-4C-alkylcarbonyloxy, trifluoromethyl, phenyl, phenyl-1-4C-alkyl, nitro, amino, 1-4C-alkoxy which is completely or partially substituted by fluorine, or is SO₂-R70 or N(R71)R72, where
R70 is 1-4C-alkyl,
R71 is hydrogen, 1-4C-alkyl, SO₂-R9 or SO₂-R10 and
R72 is 1-4C-alkyl, 1-4C-alkylcarbonyl or SO₂-R10,
R8 is hydrogen, hydroxyl, halogen, 1-4C-alkoxy or 1-4C-alkyl
and where
R9 and R10 independently of one another are 1-4C-alkyl, phenyl, phenyl-1-4C-alkyl or phenyl which is substituted by one or more identical or different substituents, where the substituents are selected from the group consisting of nitro, 1-4C-alkyl, halogen, 1-4C-alkylcarbonylamino, 1-4C-alkoxy, 1-4C-alkoxy which is completely or partially substituted by fluorine, cyano, phenyl, naphthyl, trifluoromethyl and 1-4C-alkoxycarbonyl,
or the salts of these compounds.

2. Compounds of formula I as claimed in claim 1, in which
R1 is hydroxyl, 1-4C-alkoxy, 3-7C-cycloalkoxy, 3-7C-cycloalkylmethoxy or 1-4C-alkoxy which is completely or partially substituted by fluorine,
R2 is hydroxyl, 1-4C-alkoxy, 3-7C-cycloalkoxy, 3-7C-cycloalkylmethoxy or 1-4C-alkoxy which is completely or partially substituted by fluorine,
or in which
R1 and R2 together are a 1-2C-alkylenedioxy group,
R3 is hydrogen or 1-4C-alkyl,
R31 is hydrogen or 1-4C-alkyl,
or in which
R3 and R31 together are a 1-4C-alkylene group,
R4 is hydrogen or 1-4C-alkyl,
R5 is hydrogen,
R51 is hydrogen,
or in which
R5 and R51 together are an additional bond,
R6 is a pyridyl radical which is substituted by R61, where
R61 is hydrogen, hydroxyl, halogen, 1-4C-alkoxy, 1-4C-alkyl, carboxyl, trifluoromethyl, 1-4C-alkoxycarbonyl or 1-4C-alkoxy which is completely or partially substituted by fluorine,
or the salts of these compounds.

3. Compounds of formula I as claimed in claim 1, in which
R1 is hydroxyl, 1-4C-alkoxy, 3-7C-cycloalkoxy, 3-7C-cycloalkylmethoxy or 1-4C-alkoxy which is completely or partially substituted by fluorine,
R2 is hydroxyl, 1-4C-alkoxy, 3-7C-cycloalkoxy, 3-7C-cycloalkylmethoxy or 1-4C-alkoxy which is completely or partially substituted by fluorine,
or in which
R1 and R2 together are a 1-2C-alkylenedioxy group,
R3 is hydrogen or 1-4C-alkyl,
R31 is hydrogen or 1-4C-alkyl,
or in which
R3 and R31 together are a 1-4C-alkylene group,
R4 is hydrogen or 1-4C-alkyl,
R5 is hydrogen,
R51 is hydrogen,
or in which
R5 and R51 together are an additional bond,
R6 is a phenyl radical which is substituted by R7 and R8, where
R7 is hydroxyl, halogen, cyano, 1-4C-alkyl, 1-4C-alkoxy, 1-4C-alkylcarbonyloxy, trifluoromethyl, phenyl, phenyl-1-4C-alkyl, nitro, amino or N(R71)R72, where
R71 is hydrogen, 1-4C-alkyl, SO₂-R9 or SO₂-R10 and
R72 is 1-4C-alkyl, 1-4C-alkylcarbonyl or SO₂-R10,
R8 is hydrogen, hydroxyl, halogen, 1-4C-alkoxy or 1-4C-alkyl
and where
R9 and R10 independently of one another are 1-40-alkyl, phenyl, phenyl-1-4C-alkyl or phenyl which is substituted by one or more identical or different substituents, the substituents being selected from the group consisting of nitro, 1-4C-alkyl, halogen, 1-4C-alkylcarbonylamino, 1-4C-alkoxy, 1-4C-alkoxy completely or partially substituted by fluorine, cyano, phenyl, naphthyl and trifluoromethyl,
or the salts of these compounds.

4. Compounds of formula I as claimed in claim 1, in which
R1 is hydroxyl, 1-4C-alkoxy, 3-7C-cycloalkoxy, 3-7C-cycloalkylmethoxy or 1-4C-alkoxy which is completely or partially substituted by fluorine,
R2 is hydroxyl, 1-4C-alkoxy, 3-7C-cycloalkoxy, 3-7C-cycloalkylmethoxy or 1-4C-alkoxy which is completely or partially substituted by fluorine,
or in which
R1 and R2 together are a 1-2C-alkylenedioxy group,
R3 is hydrogen or 1-4C-alkyl,
R31 is hydrogen or 1-4C-alkyl,
or in which
R3 and R31 together are a 1-4C-alkylene group,
R4 is hydrogen or 1-4C-alkyl,
R5 is hydrogen,
R51 is hydrogen,
or in which
R5 and R51 together are an additional bond,
R6 is a phenyl radical which is substituted by R7 and R8, where
R7 is hydroxyl, halogen, cyano, 1-4C-alkyl, 1-4C-alkoxy, 1-4C-alkylcarbonyloxy, trifluoromethyl, phenyl, phenyl-1-4C-alkyl, nitro, amino, 1-4C-alkoxy which is completely or partially substituted by fluorine, or is SO₂-R70 or N(R71)R72, where
R70 is 1-4C-alkyl,
R71 is hydrogen, 1-4C-alkyl, SO₂-R9 or SO₂-R10 and
R72 is 1-4C-alkyl, 1-4C-alkylcarbonyl or SO₂-R10,
R8 is hydrogen, hydroxyl, halogen, 1-4C-alkoxy or 1-4C-alkyl
and where
R9 and R10 independently of one another are 1-4C-alkyl, phenyl, phenyl-1-4C-alkyl or phenyl which is substituted by one or more identical or different substituents, the substituents being selected from the group consisting of nitro, 1-4C-alkyl, halogen, 1-4C-alkylcarbonylamino, 1-4C-alkoxy, 1-4C-alkoxy which is completely or partially substituted by fluorine, cyano, phenyl, naphthyl, trifluoromethyl and 1-4C-alkoxycarbonyl,
or the salts of these compounds.

5. Compounds of formula I as claimed in claim 1, in which
R1 is 1-4C-alkoxy or 3-7C-cycloalkoxy,
R2 is 1-4C-alkoxy or 3-7C-cycloalkoxy,
R3 is hydrogen,
R31 is hydrogen,
or in which
R3 and R31 together are a 1-2C-alkylene group,
R4 is hydrogen or 1-4C-alkyl,
R5 is hydrogen,
R51 is hydrogen,
or in which
R5 and R51 together are an additional bond,
R6 is a phenyl radical which is substituted by R7 and R8, where
R7 is hydroxyl, halogen, cyano, 1-4C-alkoxy, 1-4C-alkylcarbonyloxy, trifluoromethyl, phenyl, phenyl-1-4C-alkyl, nitro, amino, 1-4C-alkoxy which is completely or partially substituted by fluorine, or is SO₂-R70 or N(R71)R72, where
R70 is 1-4C-alkyl,
R71 is hydrogen, 1-4C-alkyl or SO₂-R10 and
R72 is 1-4C-alkylcarbonyl or SO₂-R10,
R8 is hydrogen, halogen or 1-4C-alkoxy
and where
R10 is phenyl which is substituted by a substituent, the substituent being selected from the group consisting of nitro, 1-4C-alkyl and 1-4C-alkoxycarbonyl,
or the salts of these compounds.

6. A medicament comprising at least one compound of formula I as claimed in claim 1 together with pharmaceutical auxiliaries and/or excipients.

7. The use of compounds of formula I as claimed in claim 1 for the production of medicaments for the treatment of airway disorders.

## Revendications

1. Composé de formule (I) 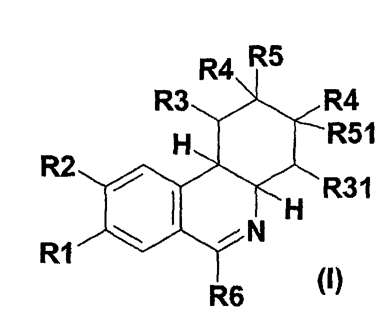 dans laquelle
R1 représente un groupe hydroxyle, alcoxy en C1-4, cycloalcoxy en C3-7, (cycloalkyle en C3-7)-méthoxy ou alcoxy en C1-4 totalement ou partiellement fluoré,
R2 représente un groupe hydroxyle, alcoxy en C1-4, cycloalcoxy en C3-7, (cycloalkyle en C3-7)-méthoxy ou alcoxy en C1-4 totalement ou partiellement fluoré, ou
R1 et R2 représentent conjointement un groupe alkylènedioxy en C1-2,
R3 représente un atome d'hydrogène ou un groupe alkyle en C1-4,
R31 représente un atome d'hydrogène ou un groupe alkyle en C1-4, ou
R3 et R31 représentent conjointement un groupe alkylène en C1-4,
R4 représente un atome d'hydrogène ou un groupe alkyle en C1-4,
R5 représente un atome d'hydrogène,
R51 représente un atome d'hydrogène, ou
R5 et R51 représentent conjointement une liaison supplémentaire,
R6 représente un groupe pyridyle portant un substituant R61 ou un groupe phényle portant des substituants R7 et R8, où
R61 représente un atome d'hydrogène ou un groupe hydroxyle, halogéno, alcoxy en C1-4, alkyle en C1-4, carboxyle, trifluorométhyle, (alkyle en C1-4)oxycarbonyle ou alcoxy en C1-4 totalement ou partiellement fluoré,
R7 représente un groupe hydroxy, halogéno, cyano, alkyle en C1-4, alcoxy en C1-4, (alkyle en C1-4)carbonyloxy, trifluorométhyle, phényle, phényl-(alkyle en C1-4), nitro, amino, alcoxy en C1-4 totalement ou partiellement fluoré, SO2R70 ou N(R71)(R72) où
R70 représente un groupe alkyle en C1-4,
R71 représente un atome d'hydrogène, un groupe alkyle en C1-4, SO2-R9 ou SO2-R10 et
R72 représente un groupe alkyle en C1-4, (alkyle en C1-4)carbonyle ou SO2-R10,
R8 représente un atome d'hydrogène, un groupe hydroxyle, halogéno, alcoxy en C1-4 ou alkyle en C1-4, et où
R9 et R10 représentent, indépendamment l'un de l'autre, un groupe alkyle en C1-4, phényle, phényl(alkyle en C1-4) ou phényle portant un ou plusieurs substituants, identiques ou différents, lesdits substituants étant choisis parmi les groupes nitro, alkyle en C1-4, halogéno, (alkyle en C1-4)carbonylamino, alcoxy en C1-4, alcoxy en C1-4 totalement ou partiellement fluoré, cyano, phényle, naphtyle, trifluorométhyle ou (alcoxy en C1-4)carbonyle,
ou un sel d'un tel composé.

2. Composé de formule (I) selon la revendication 1, dans lequel
R1 représente un groupe hydroxyle, alcoxy en C1-4, cycloalcoxy en C3-7, (cycloalkyle en C3-7)-méthoxy ou alcoxy en C1-4 totalement ou partiellement fluoré,
R2 représente un groupe hydroxyle, alcoxy en C1-4, cycloalcoxy en C3-7, (cycloalkyle en C3-7)-méthoxy ou alcoxy en C1-4 totalement ou partiellement fluoré, ou
R1 et R2 représentent conjointement un groupe alkylènedioxy en C1-2,
R3 représente un atome d'hydrogène ou un groupe alkyle en C1-4,
R31 représente un atome d'hydrogène ou un groupe alkyle en C1-4, ou
R3 et R31 représentent conjointement un groupe alkylène en C1-4,
R4 représente un atome d'hydrogène ou un groupe alkyle en C1-4,
R5 représente un atome d'hydrogène,
R51 représente un atome d'hydrogène, ou
R5 et R51 représentent conjointement une liaison supplémentaire,
R6 représente un groupe pyridyle portant un substituant R61, où
R61 représente un atome d'hydrogène ou un groupe hydroxyle, halogéno, alcoxy en C1-4, alkyle en C1-4, carboxyle, trifluorométhyle, (alkyle en C1-4)oxycarbonyle ou alcoxy en C1-4 totalement ou partiellement fluoré,
ou un sel d'un tel composé.

3. Composé de formule (I) selon la revendication 1, dans lequel
R1 représente un groupe hydroxyle, alcoxy en C1-4, cycloalcoxy en C3-7, (cycloalkyle en C3-7)-méthoxy ou alcoxy en C1-4 totalement ou partiellement fluoré,
R2 représente un groupe hydroxyle, alcoxy en C1-4, cycloalcoxy en C3-7, (cycloalkyle en C3-7)-méthoxy ou alcoxy en C1-4 totalement ou partiellement fluoré, ou
R1 et R2 représentent conjointement un groupe alkylènedioxy en C1-2,
R3 représente un atome d'hydrogène ou un groupe alkyle en C1-4,
R31 représente un atome d'hydrogène ou un groupe alkyle en C1-4, ou
R3 et R31 représentent conjointement un groupe alkylène en C1-4,
R4 représente un atome d'hydrogène ou un groupe alkyle en C1-4,
R5 représente un atome d'hydrogène,
R51 représente un atome d'hydrogène, ou
R5 et R51 représentent conjointement une liaison supplémentaire,
R6 représente un groupe phényle portant des substituants R7 et R8, où
R7 représente un groupe hydroxy, halogéno, cyano, alkyle en C1-4, alcoxy en C1-4, (alkyle en C1-4)carbonyloxy, trifluorométhyle, phényle, phényl-(alkyle en C1-4), nitro, amino ou N(R71)R72, où
R71 représente un atome d'hydrogène, un groupe alkyle en C1-4, SO2-R9 ou SO2R10 et
R72 représente un groupe alkyle en C1-4, (alkyle en C1-4)-carbonyle ou SO2-R10, et
R8 représente un atome d'hydrogène, un groupe hydroxyle, halogéno, alcoxy en C1-4 ou alkyle en C1-4, et où
R9 et R10 représentent, indépendamment l'un de l'autre, un groupe alkyle en C1-4, phényle, phényl(alkyle en C1-4) ou phényle portant un ou plusieurs substituants identiques ou différents, lesdits substituants étant choisis parmi les groupes nitro, alkyle en C1-4, halogéno, (alkyle en _C₁-4)carbonylamino, alcoxy en C1-4, ou alcoxy en C1-4 totalement ou partiellement fluoré, cyano, phényle, naphtyle ou trifluorométhyle,
ou un sel d'un tel composé.

4. Composé de formule (I) selon la revendication 1, dans lequel
R1 représente un groupe hydroxyle, alcoxy en C1-4, cycloalcoxy en C3-7, (cycloalkyle en C3-7)-méthoxy ou alcoxy en C1-4 totalement ou partiellement fluoré,
R2 représente un groupe hydroxyle, alcoxy en C1-4, cycloalcoxy en C3-7, (cycloalkyle en C3-7)-méthoxy ou alcoxy en C1-4 totalement ou partiellement fluoré,
ou R1 et R2 représentent conjointement un groupe alkylènedioxy en C1-2,
R3 représente un atome d'hydrogène ou un groupe alkyle en C1-4, R31 représente un atome d'hydrogène ou un groupe alkyle en C1-4, ou
R3 et R31 représentent conjointement un groupe alkylène en C1-4,
R4 représente un atome d'hydrogène ou un groupe alkyle en C1-4,
R5 représente un atome d'hydrogène,
R51 représente un atome d'hydrogène, ou
R5 et R51 représentent conjointement une liaison supplémentaire,
R6 représente un groupe phényle portant des substituants R7 et R8, où
R7 représente un groupe hydroxyle, halogéno, cyano, alkyle en C1-4, alcoxy en C1-4, (alkyle en C1-4)-carbonyloxy, trifluorométhyle, phényle, phényl-(alkyle en C1-4), nitro, amino, alcoxy en C1-4 totalement ou partiellement fluoré, SO2R70 ou N(R71)(R72), où
R70 représente un groupe alkyle en C1-4,
R71 représente un atome d'hydrogène, un groupe alkyle en C1-4, SO2-R9 ou SO2-R10 et
R72 représente un groupe alkyle en C1-4, (alkyle en C1-4)carbonyle ou SO2-R10,
R8 représente un atome d'hydrogène, un groupe hydroxyle, halogéno, alcoxy en C1-4 ou alkyle en C1-4, et où
R9 et R10 représentent, indépendamment l'un de l'autre, un groupe alkyle en C1-4, phényle, phényl(alkyle en C1-4) ou phényle portant un ou plusieurs substituants identiques ou différents, lesdits substituants étant choisis parmi les groupes nitro, alkyle en C1-4, halogéno, (alkyle en C1-4)carbonylamino, alcoxy en C1-4, alcoxy en C1-4 totalement ou partiellement fluoré, cyano, phényle, naphtyle, trifluorométhyle ou (alcoxy en C1-4)carbonyle,
ou un sel d'un tel composé.

5. Composé de formule (I) selon la revendication 1, dans lequel
R1 représente un groupe alcoxy en C1-4 ou cycloalcoxy en C3-7,
R2 représente un groupe alcoxy en C1-4 ou cycloalcoxy en C3-7,
R3 représente un atome d'hydrogène,
R31 représente un atome d'hydrogène, ou
R3 et R31 représentent conjointement un groupe alkylène en C1-2,
R4 eprésente un atome d'hydrogène ou un groupe alkyle en C1-4,
R5 représente un atome d'hydrogène,
R51 représente un atome d'hydrogène, ou
R5 et R51 représentent conjointement une liaison supplémentaire,
R6 représente un groupe phényle portant des substituants R7 et R8, où
R7 représente un groupe hydroxy, halogéno, cyano, alcoxy en C1-4, (alkyle en C1-4)carbonyloxy, trifluorométhyle, phényle, phényl-(alkyle en C1-4), nitro, amino, alcoxy en C1-4 totalement ou partiellement fluoré, SO2R70 ou N(R71)(R72) où
R70 représente un groupe alkyle en C1-4,
R71 représente un atome d'hydrogène, un groupe alkyle en C1-4 ou SO2-R10 et
R72 représente un groupe (alkyle en C1-4)carbonyle ou SO2-R10,
R8 représente un atome d'hydrogène ou d'halogène ou un groupe alcoxy en C1-4, et où
R10 représente un groupe phényle portant un substituant, ledit substituant étant choisi parmi les groupes nitro, alkyle en C1-4 ou (alcoxy en C1-4)carbonyle,
ou un sel d'un tel composé.

6. Médicament comprenant au moins un composé de formule (I) selon la revendication 1 avec des adjuvants et/ou excipients pharmaceutiques.

7. Utilisation de composés de formule (I) selon la revendication 1 pour la fabrication d'un médicament destiné au traitement de désordres des voies respiratoires.
